# NEW EUROPEAN PATENT SPECIFICATION

(11) **EP 2 227 488 B3**
(45) Date of publication of this specification: **19.11.2014**
(45) Mention of the grant of the patent: 22.01.2014
(21) Application number: 08855772.3
(22) Date of filing: 03.12.2008
(51) Int. Cl.: C07K 14/755, A61K 38/37

(54) **FVIII PEPTIDES AND THEIR USE IN TOLERISING HAEMOPHILIACS**
TOLERANZ-INDUZIERENDE FVIII PEPTIDEN UND DEREN VERWENDUNG IN DER BEHANDLUNG VON HÄMOPHILIE
PEPTIDES FVIII ET LEUR UTILISATION DANS LA TOLÉRISATION DE L'HÉMOPHILIE

(30) Priority: 04.12.2007 GB 0723712
(43) Date of publication of application: 15.09.2010
(73) Proprietor: Apitope International NV, 3590 Diepenbeek (BE)
(72) Inventor: WRAITH, David, Bristol BS1 5UB (GB)
(74) Representative: Holliday, Louise Caroline
(86) International application number: PCT/GB2008/003996
(87) International publication number: WO 2009/071886

(56) References cited:
- WO-A-02/16410
- WO-A-90/15615
- WO-A-02/060917
- WO-A-02/098454
- WO-A-03/087161
- JONES T D ET AL: "Identification and removal of a promiscuous CD4+ T cell epitope from the C1 domain of factor VIII." JOURNAL OF THROMBOSIS AND HAEMOSTASIS : JTH MAY 2005, vol. 3, no. 5, May 2005 (2005-05), pages 991-1000, XP002518538 ISSN: 1538-7933
- SUGIHARA T ET AL: "IDENTIFICATION OF PLASMA ANTIBODY EPITOPES AND GENE ABNORMALITIES IN JAPANESE HEMOPHILIA A PATIENTS WITH FACTOR VIII INHIBITOR" NAGOYA JOURNAL OF MEDICAL SCIENCE, NAGOYA DAIGAKU IGAKUBU, NAGOYA, JP, vol. 63, no. 1/02, 1 May 2000 (2000-05-01), pages 25-39, XP008014431 ISSN: 0027-7622

### SEQUENCE LISTING

<110> Apitope Technology (Bristol) Limited
<120> PEPTIDES
<130> P031150WO
<150> GB 0723712.6
   <151> 2007-12-04
<160> 96
<170> Patentln version 3.5
<210> 1
   <211> 2351
   <212> PRT
   <213> Homo sapiens
   <400> 1
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
   180 185 190
   Gly Ala Leu Leu Val Cys Arg Glu Gly Ser Leu Ala Lys Glu Lys Thr
   195 200 205
   Gln Thr Leu His Lys Phe Ile Leu Leu Phe Ala Val Phe Asp Glu Gly
   210 215 220
   Lys Ser Trp His Ser Glu Thr Lys Asn Ser Leu Met Gln Asp Arg Asp
   225 230 235 240
   Ala Ala Ser Ala Arg Ala Trp Pro Lys Met His Thr Val Asn Gly Tyr
   245 250 255
   Val Asn Arg Ser Leu Pro Gly Leu Ile Gly Cys His Arg Lys Ser Val
   260 265 270
   Tyr Trp His Val Ile Gly Met Gly Thr Thr Pro Glu Val His Ser Ile
   275 280 285
   Phe Leu Glu Gly His Thr Phe Leu Val Arg Asn His Arg Gln Ala Ser
   290 295 300
   Leu Glu Ile Ser Pro Ile Thr Phe Leu Thr Ala Gln Thr Leu Leu Met
   305 310 315 320
   Asp Leu Gly Gln Phe Leu Leu Phe Cys His Ile Ser Ser His Gln His
   325 330 335
   Asp Gly Met Glu Ala Tyr Val Lys Val Asp Ser Cys Pro Glu Glu Pro
   340 345 350
   Gln Leu Arg Met Lys Asn Asn Glu Glu Ala Glu Asp Tyr Asp Asp Asp
   355 360 365
   Leu Thr Asp Ser Glu Met Asp Val Val Arg Phe Asp Asp Asp Asn Ser
   370 375 380
   Pro Ser Phe Ile Gln Ile Arg Ser Val Ala Lys Lys His Pro Lys Thr
   385 390 395 400
   Trp Val His Tyr Ile Ala Ala Glu Glu Glu Asp Trp Asp Tyr Ala Pro
   405 410 415
   Leu Val Leu Ala Pro Asp Asp Arg Ser Tyr Lys Ser Gln Tyr Leu Asn
   420 425 430
   Asn Gly Pro Gln Arg Ile Gly Arg Lys Tyr Lys Lys Val Arg Phe Met
           435                 440                 445
   Ala Tyr Thr Asp Glu Thr Phe Lys Thr Arg Glu Ala Ile Gln His Glu
       450                 455                 460
   Ser Gly Ile Leu Gly Pro Leu Leu Tyr Gly Glu Val Gly Asp Thr Leu
   465                 470                 475                 480
   Leu Ile Ile Phe Lys Asn Gln Ala Ser Arg Pro Tyr Asn Ile Tyr Pro
                   485                 490                 495
   His Gly Ile Thr Asp Val Arg Pro Leu Tyr Ser Arg Arg Leu Pro Lys
               500                 505                 510
   Gly Val Lys His Leu Lys Asp Phe Pro Ile Leu Pro Gly Glu Ile Phe
           515                 520                 525
   Lys Tyr Lys Trp Thr Val Thr Val Glu Asp Gly Pro Thr Lys Ser Asp
       530                 535                 540
   Pro Arg Cys Leu Thr Arg Tyr Tyr Ser Ser Phe Val Asn Met Glu Arg
   545                 550                 555                 560
   Asp Leu Ala Ser Gly Leu Ile Gly Pro Leu Leu Ile Cys Tyr Lys Glu
                   565                 570                 575
   Ser Val Asp Gln Arg Gly Asn Gln Ile Met Ser Asp Lys Arg Asn Val
               580                 585                 590
   Ile Leu Phe Ser Val Phe Asp Glu Asn Arg Ser Trp Tyr Leu Thr Glu
           595                 600                 605
   Asn Ile Gln Arg Phe Leu Pro Asn Pro Ala Gly Val Gln Leu Glu Asp
       610                 615                 620
   Pro Glu Phe Gln Ala Ser Asn Ile Met His Ser Ile Asn Gly Tyr Val
   625                 630                 635                 640
   Phe Asp Ser Leu Gln Leu Ser Val Cys Leu His Glu Val Ala Tyr Trp
                   645                 650                 655
   Tyr Ile Leu Ser Ile Gly Ala Gln Thr Asp Phe Leu Ser Val Phe Phe
               660                 665                 670
   Ser Gly Tyr Thr Phe Lys His Lys Met Val Tyr Glu Asp Thr Leu Thr
           675                 680                 685
   Leu Phe Pro Phe Ser Gly Glu Thr Val Phe Met Ser Met Glu Asn Pro
       690                 695                 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Asn Ser Arg His Pro
           755                 760                 765
   Ser Thr Arg Gln Lys Gln Phe Asn Ala Thr Thr Ile Pro Glu Asn Asp
       770                 775                 780
   Ile Glu Lys Thr Asp Pro Trp Phe Ala His Arg Thr Pro Met Pro Lys
   785                 790                 795                 800
   Ile Gln Asn Val Ser Ser Ser Asp Leu Leu Met Leu Leu Arg Gln Ser
                   805                 810                 815
   Pro Thr Pro His Gly Leu Ser Leu Ser Asp Leu Gln Glu Ala Lys Tyr
               820                 825                 830
   Glu Thr Phe Ser Asp Asp Pro Ser Pro Gly Ala Ile Asp Ser Asn Asn
           835                 840                 845
   Ser Leu Ser Glu Met Thr His Phe Arg Pro Gln Leu His His Ser Gly
       850                 855                 860
   Asp Met Val Phe Thr Pro Glu Ser Gly Leu Gln Leu Arg Leu Asn Glu
   865                 870                 875                 880
   Lys Leu Gly Thr Thr Ala Ala Thr Glu Leu Lys Lys Leu Asp Phe Lys
                   885                 890                 895
   Val Ser Ser Thr Ser Asn Asn Leu Ile Ser Thr Ile Pro Ser Asp Asn
               900                 905                 910
   Leu Ala Ala Gly Thr Asp Asn Thr Ser Ser Leu Gly Pro Pro Ser Met
           915                 920                 925
   Pro Val His Tyr Asp Ser Gln Leu Asp Thr Thr Leu Phe Gly Lys Lys
       930                 935                 940
   Ser Ser Pro Leu Thr Glu Ser Gly Gly Pro Leu Ser Leu Ser Glu Glu
   945                 950                 955                 960
   Asn Asn Asp Ser Lys Leu Leu Glu Ser Gly Leu Met Asn Ser Gln Glu
                   965                 970                 975
   Ser Ser Trp Gly Lys Asn Val Ser Ser Thr Glu Ser Gly Arg Leu Phe
               980                 985                 990
   Lys Gly Lys Arg Ala His Gly Pro Ala Leu Leu Thr Lys Asp Asn Ala
           995                 1000                1005
   Leu Phe Lys Val Ser Ile Ser Leu Leu Lys Thr Asn Lys Thr Ser
       1010                1015                1020
   Asn Asn Ser Ala Thr Asn Arg Lys Thr His Ile Asp Gly Pro Ser
       1025                1030                1035
   Leu Leu Ile Glu Asn Ser Pro Ser Val Trp Gln Asn Ile Leu Glu
       1040                1045                1050
   Ser Asp Thr Glu Phe Lys Lys Val Thr Pro Leu Ile His Asp Arg
       1055                1060                1065
   Met Leu Met Asp Lys Asn Ala Thr Ala Leu Arg Leu Asn His Met
       1070                1075                1080
   Ser Asn Lys Thr Thr Ser Ser Lys Asn Met Glu Met Val Gln Gln
       1085                1090                1095
   Lys Lys Glu Gly Pro Ile Pro Pro Asp Ala Gln Asn Pro Asp Met
       1100                1105                1110
   Ser Phe Phe Lys Met Leu Phe Leu Pro Glu Ser Ala Arg Trp Ile
       1115                1120                1125
   Gln Arg Thr His Gly Lys Asn Ser Leu Asn Ser Gly Gln Gly Pro
       1130                1135                1140
   Ser Pro Lys Gln Leu Val Ser Leu Gly Pro Glu Lys Ser Val Glu
       1145                1150                1155
   Gly Gln Asn Phe Leu Ser Glu Lys Asn Lys Val Val Val Gly Lys
       1160                1165                1170
   Gly Glu Phe Thr Lys Asp Val Gly Leu Lys Glu Met Val Phe Pro
       1175                1180                1185
   Ser Ser Arg Asn Leu Phe Leu Thr Asn Leu Asp Asn Leu His Glu
   1190 1195 1200
   Asn Asn Thr His Asn Gln Glu Lys Lys Ile Gln Glu Glu Ile Glu
   1205 1210 1215
   Lys Lys Glu Thr Leu Ile Gln Glu Asn Val Val Leu Pro Gln Ile
   1220 1225 1230
   His Thr Val Thr Gly Thr Lys Asn Phe Met Lys Asn Leu Phe Leu
   1235 1240 1245
   Leu Ser Thr Arg Gln Asn Val Glu Gly Ser Tyr Asp Gly Ala Tyr
   1250 1255 1260
   Ala Pro Val Leu Gln Asp Phe Arg Ser Leu Asn Asp Ser Thr Asn
   1265 1270 1275
   Arg Thr Lys Lys His Thr Ala His Phe Ser Lys Lys Gly Glu Glu
   1280 1285 1290
   Glu Asn Leu Glu Gly Leu Gly Asn Gln Thr Lys Gln Ile Val Glu
   1295 1300 1305
   Lys Tyr Ala Cys Thr Thr Arg Ile Ser Pro Asn Thr Ser Gln Gln
   1310 1315 1320
   Asn Phe Val Thr Gln Arg Ser Lys Arg Ala Leu Lys Gln Phe Arg
   1325 1330 1335
   Leu Pro Leu Glu Glu Thr Glu Leu Glu Lys Arg Ile Ile Val Asp
   1340 1345 1350
   Asp Thr Ser Thr Gln Trp Ser Lys Asn Met Lys His Leu Thr Pro
   1355 1360 1365
   Ser Thr Leu Thr Gln Ile Asp Tyr Asn Glu Lys Glu Lys Gly Ala
   1370 1375 1380
   Ile Thr Gln Ser Pro Leu Ser Asp Cys Leu Thr Arg Ser His Ser
   1385 1390 1395
   Ile Pro Gln Ala Asn Arg Ser Pro Leu Pro Ile Ala Lys Val Ser
   1400 1405 1410
   Ser Phe Pro Ser Ile Arg Pro Ile Tyr Leu Thr Arg Val Leu Phe
   1415 1420 1425
   Gln Asp Asn Ser Ser His Leu Pro Ala Ala Ser Tyr Arg Lys Lys
   1430 1435 1440
   Asp Ser Gly Val Gln Glu Ser Ser His Phe Leu Gln Gly Ala Lys
   1445 1450 1455
   Lys Asn Asn Leu Ser Leu Ala Ile Leu Thr Leu Glu Met Thr Gly
   1460 1465 1470
   Asp Gln Arg Glu Val Gly Ser Leu Gly Thr Ser Ala Thr Asn Ser
   1475 1480 1485
   Val Thr Tyr Lys Lys Val Glu Asn Thr Val Leu Pro Lys Pro Asp
   1490 1495 1500
   Leu Pro Lys Thr Ser Gly Lys Val Glu Leu Leu Pro Lys Val His
   1505 1510 1515
   Ile Tyr Gln Lys Asp Leu Phe Pro Thr Glu Thr Ser Asn Gly Ser
   1520 1525 1530
   Pro Gly His Leu Asp Leu Val Glu Gly Ser Leu Leu Gln Gly Thr
   1535 1540 1545
   Glu Gly Ala Ile Lys Trp Asn Glu Ala Asn Arg Pro Gly Lys Val
   1550 1555 1560
   Pro Phe Leu Arg Val Ala Thr Glu Ser Ser Ala Lys Thr Pro Ser
   1565 1570 1575
   Lys Leu Leu Asp Pro Leu Ala Trp Asp Asn His Tyr Gly Thr Gln
   1580 1585 1590
   Ile Pro Lys Glu Glu Trp Lys Ser Gln Glu Lys Ser Pro Glu Lys
   1595 1600 1605
   Thr Ala Phe Lys Lys Lys Asp Thr Ile Leu Ser Leu Asn Ala Cys
   1610 1615 1620
   Glu Ser Asn His Ala Ile Ala Ala Ile Asn Glu Gly Gln Asn Lys
   1625 1630 1635
   Pro Glu Ile Glu Val Thr Trp Ala Lys Gln Gly Arg Thr Glu Arg
   1640 1645 1650
   Leu Cys Ser Gln Asn Pro Pro Val Leu Lys Arg His Gln Arg Glu
   1655 1660 1665
   Ile Thr Arg Thr Thr Leu Gln Ser Asp Gln Glu Glu Ile Asp Tyr
   1670 1675 1680
   Asp Asp Thr Ile Ser Val Glu Met Lys Lys Glu Asp Phe Asp Ile
   1685 1690 1695
   Tyr Asp Glu Asp Glu Asn Gln Ser Pro Arg Ser Phe Gln Lys Lys
   1700 1705 1710
   Thr Arg His Tyr Phe Ile Ala Ala Val Glu Arg Leu Trp Asp Tyr
   1715 1720 1725
   Gly Met Ser Ser Ser Pro His Val Leu Arg Asn Arg Ala Gln Ser
   1730 1735 1740
   Gly Ser Val Pro Gln Phe Lys Lys Val Val Phe Gln Glu Phe Thr
   1745 1750 1755
   Asp Gly Ser Phe Thr Gln Pro Leu Tyr Arg Gly Glu Leu Asn Glu
   1760 1765 1770
   His Leu Gly Leu Leu Gly Pro Tyr Ile Arg Ala Glu Val Glu Asp
   1775 1780 1785
   Asn Ile Met Val Thr Phe Arg Asn Gln Ala Ser Arg Pro Tyr Ser
   1790 1795 1800
   Phe Tyr Ser Ser Leu Ile Ser Tyr Glu Glu Asp Gln Arg Gln Gly
   1805 1810 1815
   Ala Glu Pro Arg Lys Asn Phe Val Lys Pro Asn Glu Thr Lys Thr
   1820 1825 1830
   Tyr Phe Trp Lys Val Gln His His Met Ala Pro Thr Lys Asp Glu
   1835 1840 1845
   Phe Asp Cys Lys Ala Trp Ala Tyr Phe Ser Asp Val Asp Leu Glu
   1850 1855 1860
   Lys Asp Val His Ser Gly Leu Ile Gly Pro Leu Leu Val Cys His
   1865 1870 1875
   Thr Asn Thr Leu Asn Pro Ala His Gly Arg Gln Val Thr Val Gln
   1880 1885 1890
   Glu Phe Ala Leu Phe Phe Thr Ile Phe Asp Glu Thr Lys Ser Trp
   1895 1900 1905
   Tyr Phe Thr Glu Asn Met Glu Arg Asn Cys Arg Ala Pro Cys Asn
   1910 1915 1920
   Ile Gln Met Glu Asp Pro Thr Phe Lys Glu Asn Tyr Arg Phe His
   1925 1930 1935
   Ala Ile Asn Gly Tyr Ile Met Asp Thr Leu Pro Gly Leu Val Met
   1940 1945 1950
   Ala Gln Asp Gln Arg Ile Arg Trp Tyr Leu Leu Ser Met Gly Ser
   1955 1960 1965
   Asn Glu Asn Ile His Ser Ile His Phe Ser Gly His Val Phe Thr
   1970 1975 1980
   Val Arg Lys Lys Glu Glu Tyr Lys Met Ala Leu Tyr Asn Leu Tyr
   1985 1990 1995
   Pro Gly Val Phe Glu Thr Val Glu Met Leu Pro Ser Lys Ala Gly
   2000 2005 2010
   Ile Trp Arg Val Glu Cys Leu Ile Gly Glu His Leu His Ala Gly
   2015 2020 2025
   Met Ser Thr Leu Phe Leu Val Tyr Ser Asn Lys Cys Gln Thr Pro
   2030 2035 2040
   Leu Gly Met Ala Ser Gly His Ile Arg Asp Phe Gln Ile Thr Ala
   2045 2050 2055
   Ser Gly Gln Tyr Gly Gln Trp Ala Pro Lys Leu Ala Arg Leu His
   2060 2065 2070
   Tyr Ser Gly Ser Ile Asn Ala Trp Ser Thr Lys Glu Pro Phe Ser
   2075 2080 2085
   Trp Ile Lys Val Asp Leu Leu Ala Pro Met Ile Ile His Gly Ile
   2090 2095 2100
   Lys Thr Gln Gly Ala Arg Gln Lys Phe Ser Ser Leu Tyr Ile Ser
   2105 2110 2115
   Gln Phe Ile Ile Met Tyr Ser Leu Asp Gly Lys Lys Trp Gln Thr
   2120 2125 2130
   Tyr Arg Gly Asn Ser Thr Gly Thr Leu Met Val Phe Phe Gly Asn
   2135 2140 2145
   Val Asp Ser Ser Gly Ile Lys His Asn Ile Phe Asn Pro Pro Ile
   2150 2155 2160
   Ile Ala Arg Tyr Ile Arg Leu His Pro Thr His Tyr Ser Ile Arg
   2165 2170 2175
   Ser Thr Leu Arg Met Glu Trp Met Gly Cys Asp Leu Asn Ser Cys
   2180 2185 2190
   Ser Met Pro Leu Gly Met Glu Ser Lys Ala Ile Ser Asp Ala Gln
   2195 2200 2205
   Ile Thr Ala Ser Ser Tyr Phe Thr Asn Met Phe Ala Thr Trp Ser
   2210 2215 2220
   Pro Ser Lys Ala Arg Leu His Leu Gln Gly Arg Ser Asn Ala Trp
   2225 2230 2235
   Arg Pro Gln Val Asn Asn Pro Lys Glu Trp Leu Gln Val Asp Phe
   2240 2245 2250
   Gln Lys Thr Met Lys Val Thr Gly Val Thr Thr Gln Gly Val Lys
   2255 2260 2265
   Ser Leu Leu Thr Ser Met Tyr Val Lys Glu Phe Leu Ile Ser Ser
   2270 2275 2280
   Ser Gln Asp Gly His Gln Trp Thr Leu Phe Phe Gln Asn Gly Lys
   2285 2290 2295
   Val Lys Val Phe Gln Gly Asn Gln Asp Ser Phe Thr Pro Val Val
   2300 2305 2310
   Asn Ser Leu Asp Pro Pro Leu Leu Thr Arg Tyr Leu Arg Ile His
   2315 2320 2325
   Pro Gln Ser Trp Val His Gln Ile Ala Leu Arg Met Glu Val Leu
   2330 2335 2340
   Gly Cys Glu Ala Gln Asp Leu Tyr
   2345 2350
<210> 2
   <211> 9
   <212> PRT
   <213> Artificial Sequence
<220>
<223> Synthetic peptide
<400> 2
   Leu Tyr Ile Ser Gln Phe Ile Ile Met
   1 5
<210> 3
   <211> 9
   <212> PRT
   <213> Artificial Sequence
<220>
   <223> Synthetic peptide
<400> 3
   Phe Ile Ile Met Tyr Ser Leu Asp Gly
   1 5
<210> 4
   <211> 9
   <212> PRT
   <213> Artificial Sequence
<220>
   <223> Synthetic peptide
<400> 4
   Ile Ala Arg Tyr Ile Arg Leu His Pro
   1 5
<210> 5
   <211> 9
   <212> PRT
   <213> Artificial Sequence
<220>
<223> Synthetic peptide
<400> 5
   Leu Ile Ile Phe Lys Asn Gln Ala Ser
   1 5
<210> 6
   <211> 9
   <212> PRT
   <213> Artificial Sequence
<220>
   <223> Synthetic peptide
<400> 6
   Leu Thr Arg Tyr Tyr Ser Ser Phe Val
   1 5
<210> 7
   <211> 9
   <212> PRT
   <213> Artificial Sequence
<220>
<223> Synthetic peptide
<400> 7
   Met Val Thr Phe Arg Asn Gln Ala Ser
   1 5
<210> 8
   <211> 9
   <212> PRT
   <213> Artificial Sequence
<220>
   <223> Synthetic peptide
<400> 8
   Leu Arg Ile His Pro Gln Ser Trp Val
   1 5
<210> 9
   <211> 15
   <212> PRT
   <213> Artificial Sequence
<220>
<223> Synthetic peptide
<400> 9
   Pro Arg Cys Leu Thr Arg Tyr Tyr Ser Ser Phe Val Asn Met Glu
   1 5 10 15
<210> 10
   <211> 15
   <212> PRT
   <213> Artificial Sequence
<220>
<223> Synthetic peptide
<400> 10
   Asp Asn Ile Met Val Thr Phe Arg Asn Gln Ala Ser Arg Pro Tyr
   1 5 10 15
<210> 11
   <211> 15
   <212> PRT
   <213> Artificial Sequence
<220>
   <223> Synthetic peptide
<400> 11
   Ser Leu Tyr Ile Ser Gln Phe Ile Ile Met Tyr Ser Leu Asp Gly
   1               5                   10                  15
<210> 12
   <211> 15
   <212> PRT
   <213> Artificial Sequence
<220>
   <223> Synthetic peptide
<400> 12
   Pro Pro Ile Ile Ala Arg Tyr Ile Arg Leu His Pro Thr His Tyr
   1               5                   10                  15
<210> 13
   <211> 15
   <212> PRT
   <213> Artificial Sequence
<220>
   <223> Synthetic peptide
<400> 13
   Asp Thr Leu Leu Ile Ile Phe Lys Asn Gln Ala Ser Arg Pro Tyr
   1               5                   10                  15
<210> 14
   <211> 15
   <212> PRT
   <213> Artificial Sequence
<220>
<223> Synthetic peptide
<400> 14
   Arg Tyr Leu Arg Ile His Pro Gln Ser Trp Val His Gln Ile Ala
   1               5                   10                  15
<210> 15
   <211> 16
   <212> PRT
   <213> Artificial Sequence
<220>
<223> Synthetic peptide
<400> 15
   Leu Tyr Ile Ser Gln Phe Ile Ile Met Met Tyr Ser Leu Asp Gly Lys
   1               5                   10                  15
<210> 16
   <211> 15
   <212> PRT
   <213> Artificial Sequence
<220>
<223> Synthetic peptide
<400> 16
   Arg Gln Lys Phe Ser Ser Leu Tyr Ile Ser Gln Phe Ile Ile Met
   1               5                   10                  15
<210> 17
   <211> 15
   <212> PRT
   <213> Artificial Sequence
<220>
<223> Synthetic peptide
<400> 17
   Gln Lys Phe Ser Ser Leu Tyr Ile Ser Gln Phe Ile Ile Met Tyr
   1               5                   10                  15
<210> 18
   <211> 15
   <212> PRT
   <213> Artificial Sequence
<220>
<223> Synthetic peptide
<400> 18
   Lys Phe Ser Ser Leu Tyr Ile Ser Gln Phe Ile Ile Met Tyr Ser
   1               5                   10                  15
<210> 19
   <211> 15
   <212> PRT
   <213> Artificial Sequence
<220>
<223> Synthetic peptide
<400> 19
   Phe Ser Ser Leu Tyr Ile Ser Gln Phe Ile Ile Met Tyr Ser Leu
   1 5 10 15
<210> 20
   <211> 15
   <212> PRT
   <213> Artificial Sequence
<220>
<223> Synthetic peptide
<400> 20
   Ser Ser Leu Tyr Ile Ser Gln Phe Ile Ile Met Tyr Ser Leu Asp
   1 5 10 15
<210> 21
   <211> 15
   <212> PRT
   <213> Artificial Sequence
<220>
<223> Synthetic peptide
<400> 21
   Tyr Ile Ser Gln Phe Ile Ile Met Tyr Ser Leu Asp Gly Lys Lys
   1 5 10 15
<210> 22
   <211> 15
   <212> PRT
   <213> Artificial Sequence
<220>
<223> Synthetic peptide
<400> 22
   Ile Ser Gln Phe Ile Ile Met Tyr Ser Leu Asp Gly Lys Lys Trp
   1 5 10 15
<210> 23
   <211> 15
   <212> PRT
   <213> Artificial Sequence
<220>
<223> Synthetic peptide
<400> 23
   Ser Gln Phe Ile Ile Met Tyr Ser Leu Asp Gly Lys Lys Trp Gln
   1               5                   10                  15
<210> 24
   <211> 15
   <212> PRT
   <213> Artificial Sequence
<220>
<223> Synthetic peptide
<400> 24
   Gln Phe Ile Ile Met Tyr Ser Leu Asp Gly Lys Lys Trp Gln Thr
   1               5                   10                  15
<210> 25
   <211> 15
   <212> PRT
   <213> Artificial Sequence
<220>
   <223> Synthetic peptide
<400> 25
   Phe Ile Ile Met Tyr Ser Leu Asp Gly Lys Lys Trp Gln Thr Tyr
   1               5                   10                  15
<210> 26
   <211> 15
   <212> PRT
   <213> Artificial Sequence
<220>
<223> Synthetic peptide
<400> 26
   Ile Ala Arg Tyr Ile Arg Leu His Pro Thr His Tyr Ser Ile Arg
   1               5                   10                  15
<210> 27
   <211> 15
   <212> PRT
   <213> Artificial Sequence
<220>
   <223> Synthetic peptide
<400> 27
   Ile Ile Ala Arg Tyr Ile Arg Leu His Pro Thr His Tyr Ser Ile
   1 5 10 15
<210> 28
   <211> 15
   <212> PRT
   <213> Artificial Sequence
<220>
<223> Synthetic peptide
<400> 28
   Pro Ile Ile Ala Arg Tyr Ile Arg Leu His Pro Thr His Tyr Ser
   1 5 10 15
<210> 29
   <211> 15
   <212> PRT
   <213> Artificial Sequence
<220>
<223> Synthetic peptide
<400> 29
   Asn Pro Pro Ile Ile Ala Arg Tyr Ile Arg Leu His Pro Thr His
   1 5 10 15
<210> 30
   <211> 15
   <212> PRT
   <213> Artificial Sequence
<220>
<223> Synthetic peptide
<400> 30
   Phe Asn Pro Pro Ile Ile Ala Arg Tyr Ile Arg Leu His Pro Thr
   1 5 10 15
<210> 31
   <211> 15
   <212> PRT
   <213> Artificial Sequence
<220>
<223> Synthetic peptide
<400> 31
   Ile Phe Asn Pro Pro Ile Ile Ala Arg Tyr Ile Arg Leu His Pro
   1 5 10 15
<210> 32
   <211> 15
   <212> PRT
<213> Artificial Sequence
<220>
<223> Synthetic peptide
<400> 32
   Leu Ile Ile Phe Lys Asn Gln Ala Ser Arg Pro Tyr Asn Ile Tyr
   1 5 10 15
<210> 33
   <211> 15
   <212> PRT
   <213> Artificial Sequence
<220>
<223> Synthetic peptide
<400> 33
   Leu Leu Ile Ile Phe Lys Asn Gln Ala Ser Arg Pro Tyr Asn Ile
   1 5 10 15
<210> 34
   <211> 15
   <212> PRT
   <213> Artificial Sequence
<220>
<223> Synthetic peptide
<400> 34
   Thr Leu Leu Ile Ile Phe Lys Asn Gln Ala Ser Arg Pro Tyr Asn
   1 5 10 15
<210> 35
   <211> 16
   <212> PRT
<213> Artificial Sequence
<220>
<223> Synthetic peptide
<400> 35
   Gly Asp Thr Leu Leu Leu Ile Ile Phe Lys Asn Gln Ala Ser Arg Pro
   1 5 10 15
<210> 36
   <211> 16
   <212> PRT
<213> Artificial Sequence
<220>
<223> Synthetic peptide
<400> 36
   Val Gly Asp Thr Leu Leu Leu Ile Ile Phe Lys Asn Gln Ala Ser Arg
   1 5 10 15
<210> 37
   <211> 16
   <212> PRT
   <213> Artificial Sequence
<220>
<223> Synthetic peptide
<400> 37
   Glu Val Gly Asp Thr Leu Leu Leu Ile Ile Phe Lys Asn Gln Ala Ser
   1 5 10 15
<210> 38
   <211> 15
   <212> PRT
   <213> Artificial Sequence
<220>
<223> Synthetic peptide
<400> 38
   Lys Ser Asp Pro Arg Cys Leu Thr Arg Tyr Tyr Ser Ser Phe Val
   1 5 10 15
<210> 39
   <211> 15
   <212> PRT
   <213> Artificial Sequence -
<220>
   <223> Synthetic peptide
<400> 39
   Ser Asp Pro Arg Cys Leu Thr Arg Tyr Tyr Ser Ser Phe Val Asn
   1 5 10 15
<210> 40
   <211> 15
   <212> PRT
   <213> Artificial Sequence
<220>
<223> Synthetic peptide
<400> 40
   Asp Pro Arg Cys Leu Thr Arg Tyr Tyr Ser Ser Phe Val Asn Met
   1 5 10 15
<210> 41
   <211> 15
   <212> PRT
<213> Artificial Sequence
<220>
<223> Synthetic peptide
<400> 41
   Arg Cys Leu Thr Arg Tyr Tyr Ser Ser Phe Val Asn Met Glu Arg
   1 5 10 15
<210> 42
   <211> 15
   <212> PRT
   <213> Artificial Sequence
<220>
<223> Synthetic peptide
<400> 42
   Cys Leu Thr Arg Tyr Tyr Ser Ser Phe Val Asn Met Glu Arg Asp
   1 5 10 15
<210> 43
   <211> 15
   <212> PRT
   <213> Artificial Sequence
<220>
<223> Synthetic peptide
<400> 43
   Leu Thr Arg Tyr Tyr Ser Ser Phe Val Asn Met Glu Arg Asp Leu
   1 5 10 15
<210> 44
   <211> 15
   <212> PRT
   <213> Artificial Sequence
<220>
<223> Synthetic peptide
<400> 44
   Glu Val Glu Asp Asn Ile Met Val Thr Phe Arg Asn Gln Ala Ser
   1 5 10 15
<210> 45
   <211> 15
   <212> PRT
   <213> Artificial Sequence
<220>
<223> Synthetic peptide
<400> 45
   Val Glu Asp Asn Ile Met Val Thr Phe Arg Asn Gln Ala Ser Arg
   1 5 10 15
<210> 46
   <211> 15
   <212> PRT
   <213> Artificial Sequence
<220>
<223> Synthetic peptide
<400> 46
   Glu Asp Asn Ile Met Val Thr Phe Arg Asn Gln Ala Ser Arg Pro
   1 5 10 15
<210> 47
   <211> 15
   <212> PRT
   <213> Artificial Sequence
<220>
<223> Synthetic peptide
<400> 47
   Asn Ile Met Val Thr Phe Arg Asn Gln Ala Ser Arg Pro Tyr Ser
   1 5 10 15
<210> 48
   <211> 15
   <212> PRT
   <213> Artificial Sequence
<220>
<223> Synthetic peptide
<400> 48
   Ile Met Val Thr Phe Arg Asn Gln Ala Ser Arg Pro Tyr Ser Phe
   1 5 10 15
<210> 49
   <211> 15
   <212> PRT
   <213> Artificial Sequence
<220>
<223> Synthetic peptide
<400> 49
   Met Val Thr Phe Arg Asn Gln Ala Ser Arg Pro Tyr Ser Phe Tyr
   1 5 10 15
<210> 50
   <211> 15
   <212> PRT
   <213> Artificial Sequence
<220>
<223> Synthetic peptide
<400> 50
   Leu Arg Ile His Pro Gln Ser Trp Val His Gln Ile Ala Leu Arg
   1 5 10 15
<210> 51
   <211> 15
   <212> PRT
   <213> Artificial Sequence
<220>
<223> Synthetic peptide
<400> 51
   Tyr Leu Arg Ile His Pro Gln Ser Trp Val His Gln Ile Ala Leu
   1 5 10 15
<210> 52
   <211> 15
   <212> PRT
   <213> Artificial Sequence
<220>
<223> Synthetic peptide
<400> 52
   Thr Arg Tyr Leu Arg Ile His Pro Gln Ser Trp Val His Gln Ile
   1 5 10 15
<210> 53
   <211> 15
   <212> PRT
   <213> Artificial Sequence
<220>
<223> Synthetic peptide
<400> 53
   Leu Thr Arg Tyr Leu Arg Ile His Pro Gln Ser Trp Val His Gln
   1 5 10 15
<210> 54
   <211> 15.
   <212> PRT
   <213> Artificial Sequence
<220>
<223> Synthetic peptide
<400> 54
   Leu Leu Thr Arg Tyr Leu Arg Ile His Pro Gln Ser Trp Val His
   1 5 10 15
<210> 55
   <211> 15
   <212> PRT
   <213> Artificial Sequence
<220>
<223> Synthetic peptide
<400> 55
   Pro Leu Leu Thr Arg Tyr Leu Arg Ile His Pro Gln Ser Trp Val
   1 5 10 15
<210> 56
   <211> 15
   <212> PRT
   <213> Artificial Sequence
<220>
<223> Synthetic peptide
<400> 56
   Gly Thr Leu Met Val Phe Phe Gly Asn Val Asp Ser Ser Gly Ile
   1 5 10 15
<210> 57
   <211> 15
   <212> PRT
   <213> Artificial Sequence
<220>
<223> Synthetic peptide
<400> 57
   Thr Gln Thr Leu His Lys Phe Ile Leu Leu Phe Ala Val Phe Asp
   1 5 10 15
<210> 58
   <211> 15
   <212> PRT
   <213> Artificial Sequence
<220>
<223> Synthetic peptide
<400> 58
   Pro Pro Leu Leu Thr Arg Tyr Leu Arg Ile His Pro Gln Ser Trp
   1 5 10 15
<210> 59
   <211> 15
   <212> PRT
   <213> Artificial Sequence
<220>
<223> Synthetic peptide
<400> 59
   Met His Thr Val Asn Gly Tyr Val Asn Arg Ser Leu Pro Gly Leu
   1 5 10 15
<210> 60
   <211> 15
   <212> PRT
   <213> Artificial Sequence
<220>
<223> Synthetic peptide
<400> 60
   Leu Gly Gln Phe Leu Leu Phe Cys His Ile Ser Ser His Gln His
   1 5 10 15
<210> 61
   <211> 15
   <212> PRT
   <213> Artificial Sequence
<220>
<223> Synthetic peptide
<400> 61
   Thr Glu Asn Ile Gln Arg Phe Leu Pro Asn Pro Ala Gly Val Gln
   1 5 10 15
<210> 62
   <211> 5
   <212> PRT
   <213> Artificial Sequence
<220>
<223> Synthetic peptide
<400> 62
   Gly Thr Leu Met Val
   1 5
<210> 63
   <211> 5
   <212> PRT
   <213> Artificial Sequence
<220>
<223> Synthetic peptide
<400> 63
   Thr Gln Thr Leu His
   1 5
<210> 64
   <211> 5
   <212> PRT
   <213> Artificial Sequence
<220>
<223> Synthetic peptide
<400> 64
   Ser Leu Tyr Ile Ser
   1 5
<210> 65
   <211> 5
   <212> PRT
   <213> Artificial Sequence
<220>
<223> Synthetic peptide
<400> 65
   Pro Pro Ile Ile Ala
   1 5
<210> 66
   <211> 5
   <212> PRT
   <213> Artificial Sequence
<220>
<223> Synthetic peptide
<400> 66
   Pro Pro Leu Leu Thr
   1 5
<210> 67
   <211> 5
   <212> PRT
   <213> Artificial Sequence
<220>
<223> Synthetic peptide
<400> 67
   Met His Thr Val Asn
   1 5
<210> 68
   <211> 5
   <212> PRT
   <213> Artificial Sequence
<220>
<223> Synthetic peptide
<400> 68
   Leu Gly Gln Phe Leu
   1 5
<210> 69
   <211> 5
   <212> PRT
   <213> Artificial Sequence
<220>
<223> Synthetic peptide
<400> 69
   Asp Thr Leu Leu Ile
   1 5
<210> 70
   <211> 5
   <212> PRT
   <213> Artificial Sequence
<220>
<223> Synthetic peptide
<400> 70
   Pro Arg Cys Leu Thr
   1 5
<210> 71
   <211> 5
   <212> PRT
   <213> Artificial Sequence
<220>
<223> Synthetic peptide
<400> 71
   Thr Glu Asn Ile Gln
   1 5
<210> 72
   <211> 5
   <212> PRT
   <213> Artificial Sequence
<220>
<223> Synthetic peptide
<400> 72
   Asp Asn Ile Met Val
   1 5
<210> 73
   <211> 5
   <212> PRT
   <213> Artificial Sequence
<220>
<223> Synthetic peptide
<400> 73
   Arg Tyr Leu Arg Ile
   1 5
<210> 74
   <211> 15
   <212> PRT
   <213> Artificial Sequence
<220>
<223> Synthetic peptide
<400> 74
   Tyr Gly Glu Val Gly Asp Thr Leu Leu Ile Ile Phe Lys Asn Gln
   1 5 10 15
<210> 75
   <211> 15
   <212> PRT
<213> Artificial Sequence
<220>
<223> Synthetic peptide
<400> 75
   Gly Glu Val Gly Asp Thr Leu Leu Ile Ile Phe Lys Asn Gln Ala
   1 5 10 15
<210> 76
   <211> 15
   <212> PRT
   <213> Artificial Sequence
<220>
<223> Synthetic peptide
<400> 76
   Glu Val Gly Asp Thr Leu Leu Ile Ile Phe Lys Asn Gln Ala Ser
   1 5 10 15
<210> 77
   <211> 15
   <212> PRT
   <213> Artificial Sequence
<220>
<223> Synthetic peptide
<400> 77
   Val Gly Asp Thr Leu Leu Ile Ile Phe Lys Asn Gln Ala Ser Arg
   1 5 10 15
<210> 78
   <211> 15
   <212> PRT
   <213> Artificial Sequence
<220>
<223> Synthetic peptide
<400> 78
   Gly Asp Thr Leu Leu Ile Ile Phe Lys Asn Gln Ala Ser Arg Pro
   1 5 10 15
<210> 79
   <211> 15
   <212> PRT
   <213> Artificial Sequence
<220>
<223> Synthetic peptide
<400> 79
   Ile Ile Phe Lys Asn Gln Ala Ser Arg Pro Tyr Asn Ile Tyr Pro
   1 5 10 15
<210> 80
   <211> 15
   <212> PRT
   <213> Artificial Sequence
<220>
<223> Synthetic peptide
<400> 80
   Ile Phe Lys Asn Gln Ala Ser Arg Pro Tyr Asn Ile Tyr Pro His
   1 5 10 15
<210> 81
   <211> 15
   <212> PRT
   <213> Artificial Sequence
<220>
<223> Synthetic peptide
<400> 81
   Pro Thr Lys Ser Asp Pro Arg Cys Leu Thr Arg Tyr Tyr Ser Ser
   1 5 10 15
<210> 82
   <211> 15
   <212> PRT
   <213> Artificial Sequence
<220>
<223> Synthetic peptide
<400> 82
   Thr Lys Ser Asp Pro Arg Cys Leu Thr Arg Tyr Tyr Ser Ser Phe
   1 5 10 15
<210> 83
   <211> 15
   <212> PRT
   <213> Artificial Sequence
<220>
<223> Synthetic peptide
<400> 83
   Thr Arg Tyr Tyr Ser Ser Phe Val Asn Met Glu Arg Asp Leu Ala
   1 5 10 15
<210> 84
   <211> 15
   <212> PRT
<213> Artificial Sequence
<220>
<223> Synthetic peptide
<400> 84
   Arg Tyr Tyr Ser Ser Phe Val Asn Met Glu Arg Asp Leu Ala Ser
   1 5 10 15
<210> 85
   <211> 15
   <212> PRT
   <213> Artificial Sequence
<220>
<223> Synthetic peptide
<400> 85
   Arg Ala Glu Val Glu Asp Asn Ile Met Val Thr Phe Arg Asn Gln
   1 5 10 15
<210> 86
   <211> 15
   <212> PRT
   <213> Artificial Sequence
<220>
<223> Synthetic peptide
<400> 86
   Ala Glu Val Glu Asp Asn Ile Met Val Thr Phe Arg Asn Gln Ala
   1 5 10 15
<210> 87
   <211> 15
   <212> PRT
   <213> Artificial Sequence
<220>
<223> Synthetic peptide
<400> 87
   Val Thr Phe Arg Asn Gln Ala Ser Arg Pro Tyr Ser Phe Tyr Ser
   1 5 10 15
<210> 88
   <211> 15
   <212> PRT
   <213> Artificial Sequence
<220>
<223> Synthetic peptide
<400> 88
   Thr Phe Arg Asn Gln Ala Ser Arg Pro Tyr Ser Phe Tyr Ser Ser
   1 5 10 15
<210> 89
   <211> 15
   <212> PRT
   <213> Artificial Sequence
<220>
<223> Synthetic peptide
<400> 89
   Leu Tyr Ile Ser Gln Phe Ile Ile Met Tyr Ser Leu Asp Gly Lys
   1 5 10 15
<210> 90
   <211> 15
   <212> PRT
   <213> Artificial Sequence
<220>
<223> Synthetic peptide
<400> 90
   His Asn Ile Phe Asn Pro Pro Ile Ile Ala Arg Tyr Ile Arg Leu
   1 5 10 15
<210> 91
   <211> 15
   <212> PRT
   <213> Artificial Sequence
<220>
<223> Synthetic peptide
<400> 91
   Asn Ile Phe Asn Pro Pro Ile Ile Ala Arg Tyr Ile Arg Leu His
   1 5 10 15
<210> 92
   <211> 15
   <212> PRT
<213> Artificial Sequence
<220>
<223> Synthetic peptide
<400> 92
   Ala Arg Tyr Ile Arg Leu His Pro Thr His Tyr Ser Ile Arg Ser
   1 5 10 15
<210> 93
   <211> 15
   <212> PRT
   <213> Artificial Sequence
<220>
<223> Synthetic peptide
<400> 93
   Arg Tyr Ile Arg Leu His Pro Thr His Tyr Ser Ile Arg Ser Thr
   1 5 10 15
<210> 94
   <211> 15
   <212> PRT
   <213> Artificial Sequence
<220>
<223> Synthetic peptide
<400> 94
   Arg Ile His Pro Gln Ser Trp Val His Gln Ile Ala Leu Arg Met
   1 5 10 15
<210> 95
   <211> 15
   <212> PRT
   <213> Artificial Sequence
<220>
<223> Synthetic peptide
<400> 95
   Ile His Pro Gln Ser Trp Val His Gln Ile Ala Leu Arg Met Glu
   1 5 10 15
<210> 96
   <211> 15
   <212> PRT
   <213> Artificial Sequence
<220>
<223> Synthetic peptide
<400> 96
   His Pro Gln Ser Trp Val His Gln Ile Ala Leu Arg Met Glu Val
   1 5 10 15

## Description

### FIELD OF THE INVENTION

The present invention relates to a peptide. In particular, it relates to peptides derivable from factor VIII (FVIII). The peptides can be used to reduce or prevent factor VIII inhibitor antibody formation, for example in haemophilia A treatment and acquired haemophilia.

### BACKGROUND TO THE INVENTION

### HAEMOPHILIA

Haemophilia belongs to a group of inheritable blood disorders that includes haemophilia A, haemophilia B (Christmas disease) and Von Willebrand's disease.

In haemophilia, the blood's ability to clot is severely reduced because an essential clotting factor is partly or completely missing, resulting in increased bleeding time. Haemophilia A is a deficiency of the clotting factor VIII, whereas Haemophilia B is a deficiency of clotting factor IX. In both diseases, the faulty gene is found on the X chromosome, so the conditions are X-linked. Haemophilia A is five times more common than haemophilia B.

Haemophilia is a lifelong inherited genetic condition, which affects females as carriers and males who inherit the condition. About a third of new diagnoses are where there is no previous family history. It appears world-wide and occurs in all racial groups. About 6,000 people are affected with haemophilia in the UK.

Haemophiliacs bleed for a prolonged period following injury. External injuries such as cuts and grazes do not usually pose serious problems: it is often possible to stop bleeding by applying a degree of pressure and covering the affected area (e.g with a plaster).

The main problem is internal bleeding into joints, muscles and soft tissues, which can occur spontaneously. Internal bleeding, such are haemorrhages into the brain, is very difficult to manage and can be fatal. Repeated bleeding in the joints causes acute pain and can cause arthritis and/or long-term joint damage leading to disability.

Treatment for haemophilia is usually by replacement of the missing clotting factor. In mild or moderate haemophilia injections may be given at the time a bleed occurs (on-demand therapy). However, in severe haemophilia regular prophylactic injections are given to help the blood to clot and minimise the likelihood of long term joint damage.

A potentially serious complication of coagulation factor replacement therapy for haemophilia A is the development of antibodies that neutralise the procoagulant function of factor VIII. Factor VIII inhibitors occur in approximately 25% of those with severe haemophilia A. Since patients with congenital haemophilia A can be genetically deficient in FVIII, the synthesis of inhibitors is an alloimmune response to the foreign protein administered to prevent or treat bleeding episodes.

CD4+ T cells play a central role in the immune response to FVIII. After being taken up by antigen-presenting cells (APCs), FVIII undergoes proteolytic degradation into peptide fragments (Reding et al (2006) Haemophilia 12(supp 6) 30-36). These peptides are then presented on the surface of the APC in association with MHC class II molecules. This complex is then recognised by the T cell receptor of a CD4+ cell specific for FVIII. In the presence of the appropriate costimulatory signals, this recognition ultimately causes the CD4+ cell to direct the synthesis of antibodies by B cells. WO 02/060917 provides a therapeutic method comprising the administration of at least one epitope peptide comprising a universal and/or immunodominant epitope sequence from a portion (fragment) of factor VIII to a mammal in need of such treatment.

The incidence of inhibitor formation initially increases with the number of factor VIII treatments, but appears to plateau after 50-100 exposure days. Inhibitor formation is much more common in severe haemophilia than in moderate or mild disease and some molecular defects, most clearly large deletions and nonsense mutations in the factor VIII light chain, appear to predispose to inhibitor formation. Parameters such as the concentration, type (purified or recombinant) of replacement factor, and treatment history may also affect the likelihood of antibody production.

The management of haemophilia patients with inhibitors is an ongoing challenge. Immune tolerance induction (ITI) using a desensitization technique is successful in some patients with alloantibodies against factor VIII. This therapeutic approach requires ongoing exposure to factor replacement therapy, so is a long-term strategy.

Although ITI can be successful, a significant proportion (about 30%) of patients fail to respond to ITI. Patients with high inhibitor titres are much less likely to respond to treatment. Another significant contributing factor is age at the start of commencing ITI, with greatly decreased success rates when the patient is older than 20 (Hay et al (2005) Seminars in Thrombosis and Hemostasis 32:15-21)

When ITI therapy is unsuccessful, the inhibitor generally persists for life, and because such patients are usually high-responders, it is necessary to treat episodes of bleeding with FVIII bypassing products, such as activated prothombin complex concentrates (FEIBA™), and recombinant-activated FVII. However, the use of such agents is associated with adverse events such as disseminated intravascular coagulation, acute myocardial infarction, pulmonary embolus and thromboses (Acharya and DiMichele (2006) Best Practice & Research Clinical Haematology 19:51-66).

Immunosuppressive therapy is sometimes used for patients who fail to response to ITI. Treatment includes administration of immunosuppressive drugs such as cyclophosphamide, prednisone, azathioprine and cyclosporine which non-specifically target the immune system. These treatments can have side-effects associated with general immunosuppression.

There is renewed interest on selective B cell depletion using Rituximab™, a humanised monoclonal antibody to B cell CD20 antigen. However, infusion reactions, serum sickness and opportunistic infections have occurred in some children treated with this drug (DiMichele (2007) J Thromb Haemost 5:143-50).

### ACQUIRED HAEMOPHILIA

Acquired haemophilia is a rare autoimmune condition which affects between 1 and 4 people in every million. In this condition, subjects who are not born with haemophilia develop antibodies against one of the clotting factors such as factor VIII. It is thought that pregnancy and autoimmune diseases such as rheumatoid arthritis and cancer may increase the risk of developing acquired haemophilia. Although there are differences in the underlying immune mechanisms leading to their production, the clinical manifestations of FVIII inhibitors produced in response to coagulation factor replacement therapy and those produced in acquired haemophilia are similar.

Acquired haemophiliac patients have a mortality rate that approaches 25%, partly because of the association of acquired inhibitors with severe bleeding complications. The therapy of acquired autoantibody inhibitors is based primarily on the need to control or prevent acute hemorrhagic complications, which frequently are life and limb threatening and secondarily to eradicate the autoantibody to restore normal coagulation.

Some bleeds associated with low titre autoantibody inhibitors (< 5 Bethesda Units) may be treated effectively with FVIII concentrates administered at high doses. Porcine FVIII concentrate was formerly considered a critical first-line therapy for acquired hemophilia-related bleeding since it was the only replacement therapy that provided an opportunity to actually measure.post-infusion FVIII coagulation activity levels in the laboratory. The product was removed from the marketplace in 2004 because of contamination of the porcine plasma pools by porcine parvovirus. Now, "bypassing" agents are most commonly used, but potential risks of thrombogenicity exist and there is only about 80% efficacy for each product. Plasma exchange via plasmapheresis and extracorporeal immunoadsorption may be necessary to temporarily reduce the inhibitor titer enough for bypassing agents or FVIII replacement to provide adequate hemostasis.

Eradication of autoantibody inhibitors depends on immunosuppressive measures, such as: (1) administration of corticosteroids with 30%-50% efficacy in 3-6 weeks; (2) use of cytotoxic and myelosuppressive chemotherapeutic agents, e.g., cyclophosphamide, cyclosporine, 2-chlorodeoxyadenosine; (3) immunomodulation with intravenous immunoglobulin; and (4) selective B-lymphocyte depletion with rituximab. Rituximab™ responders may require concurrent use of steroids and relapses may respond to retreatment.

Thus, all currently available methods for reducing alloantibody production associated with haemophilia A treatment, and autoantibody production in acquired haemophilia, have shortcomings. There is therefore a need for improved methods to address the issue of anti-FVIII antibodies in haemophilia A and acquired haemophilia.

The present inventors have found that it is possible to prevent FVIII inhibitor antibody formation by pre-tolerising the patient with FVIII-derived peptides.

### SUMMARY OF ASPECTS OF THE INVENTION

The present invention, therefore, relates to a peptide derivable from FVIII which is capable of inducing or restoring tolerance to FVIII.

The present inventors have identified a number of immunodominant regions of FVIII that are predicted to give rise to HLA-DR2 binding peptides (Table 1). Of these peptides, regions 545-559 and 1788-1803 of factor VIII are considered to represent the immunodominant T-cell epitope regions in the HLA-DR2 restricted T-cell response to human factor VIII. Treatment of mice with these peptides has been shown to lead to a substantial suppression of the immune response to factor VIII.

In a first aspect, the present invention provides a peptide consisting of one of the following sequences:

In a second aspect, the present invention provides a composition, such as a pharmaceutical composition comprising a peptide of the first aspect of the invention. The composition may comprise a plurality of such peptides. In particular, the composition may comprise the following peptides: PRCLTRYYSSFVNME and DNIMVTFRNQASRPY

The composition may be in the form of a kit, in which the plurality of peptides are provided separately for separate, subsequent, sequential or simultaneous administration.

The peptide or a composition of the invention may be for use in suppressing, reducing, or preventing the development of factor VIII inhibitor antibodies.

The peptide or composition may be for use in a method for treating haemophilia in a subject, the method comprising the step of administration of such a peptide or composition to the subject.

The subject may be deficient in FVIII. In particular the subject may have haemophilia A, and may be, or be about to, undergo factor VIII replacement therapy.

Alternatively the subject may have, or be at risk from contracting, acquired haemophilia.

Factor VIII inhibitors are found more frequently in individuals expressing HLA-DR2. The subject treated by the method of the invention may therefore be HLA-DR2 positive.

### DESCRIPTION OF THE FIGURES

Figure 1: Recall responses for lymph node cells (LNC) from FVIII+DR2+ mice primed with rhFVIII/CFA
   a) LNC proliferation to FVIII peptides 1-6
   b) LNC proliferation to FVIII peptides 7-12
   c) LNC proliferation to FVIII peptides 1, 3 and 11
Figure 2: Representative examples of FVIII+DR2+ T cell hybridoma clones specific for FVIII-derived peptides
Figure 3: Recall responses for LNC from FVIII-DR2+ mice primed with rhFVIII/CFA
Figure 4: Representative examples of FVIII-DR2+ T cell hydridoma clones specific for FVIII-derived peptides
Figure 5: FVIII-/- clones specific for a) DNIMV and b) PRCLT
Figure 6: Recall responses for LNC to FVIII for FVIII+DR2+ mice treated 3x i.p. with peptide prior to priming with rhFVIII/CFA.
Figure 7: Determination of the range of peptide epitopes capable of functions as apitopes using FVIII-DR2+ T cell hydridoma clones specific for FVIII-derived overlapping peptides. The original peptide is termed 0. One amino acid shift towards the N-terminal is -1, two amino acid shits towards the N-terminal is -2 etc. One shift towards the C-terminal is +1 etc.
Figure 8: Lymph node cell IFN-gamma production in response to FVIII for FVIII-DR2+ mice treated with FVIII-derived peptides PRCLT, DNIMV or a mixture of both of these.

### DETAILED DESCRIPTION

### PEPTIDE

The present invention relates to a peptide.

The term "peptide" is used in the normal sense to mean a series of residues, typically L-amino acids, connected one to the other typically by peptide bonds between the α-amino and carboxyl groups of adjacent amino acids. The term includes modified peptides and synthetic peptide analogues.

The peptide of the present invention may be made using chemical methods (Peptide Chemistry, A practical Textbook. Mikos Bodansky, Springer-Verlag, Berlin.). For example, peptides can be synthesized by solid phase techniques (Roberge JY et al (1995) Science 269: 202-204), cleaved from the resin, and purified by preparative high performance liquid chromatography (e.g., Creighton (1983) Proteins Structures And Molecular Principles, WH Freeman and Co, New York NY). Automated synthesis may be achieved, for example, using the ABI 43 1 A Peptide Synthesizer (Perkin Elmer) in accordance with the instructions provided by the manufacturer.

The peptide may alternatively be made by recombinant means, or by cleavage from factor VIII. The composition of a peptide may be confirmed by amino acid analysis or sequencing (e.g., the Edman degradation procedure).

For practical purposes, there are various other characteristics which the peptide may show. For example, the peptide may soluble at a concentration which permits its use *in vivo.* The peptide may be soluble at concentrations of up to 0.5 mg/ml, 1 mg/ml, or 5 mg/ml.

It is also important that the peptide is sufficiently stable *in vivo* to be therapeutically useful. The half-life of the peptide *in vivo* may be at least 10 minutes, 30 minutes, 4 hours, or 24 hours.

The peptide may also demonstrate good bioavailability *in vivo.* The peptide may maintain a conformation *in vivo* which enables it to bind to an MHC molecule at the cell surface without due hindrance.

### EPITOPES

In an adaptive immune response, T lymphocytes are capable of recognising internal epitopes of a protein antigen. Antigen presenting cells (APC) take up protein antigens and degrade them into short peptide fragments. A peptide may bind to a major histocompatability complex (MHC) class I or II molecule inside the cell and be carried to the cell surface. When presented at the cell surface in conjunction with an MHC molecule, the peptide may be recognised by a T cell (via the T cell receptor (TCR)), in which case the peptide is a T cell epitope.

An epitope is thus a peptide derivable from an antigen which is capable of binding to the peptide-binding groove of a MHC class I or II molecule and be recognised by a T cell.

The minimal epitope is the shortest fragment derivable from an epitope, which is capable of binding to the peptide-binding groove of a MHC class I or II molecule and be recognised by a T cell. For a given immunogenic region, it is typically possible to generate a "nested set" of overlapping peptides which act as epitopes, all of which contain the minimal epitope but differ in their flanking regions.

By the same token, it is possible to identify the minimal epitope for a particular MHC molecule:T cell combination by measuring the response to truncated peptides. For example if a response is obtained to the peptide comprising residues 1-15 in the overlapping library, sets which are truncated at both ends (i.e. 1-14, 1-13, 1-12 etc. and 2-15, 3-15, 4-15 etc.) can be used to identify the minimal epitope.

### APITOPES

The present inventors have previously determined that there is a link between the capacity of a peptide to bind to an MHC class I or II molecule and be presented to a T cell without further antigen processing, and the peptide's capacity to induce tolerance in *vivo* (WO 02/16410). If a peptide is too long to bind the peptide binding groove of an MHC molecule without further processing (e.g. trimming), or binds in an inappropriate conformation then it will not be tolerogenic *in vivo.* If, on the other hand, the peptide is of an appropriate size and conformation to bind directly to the MHC peptide binding groove and be presented to a T cell, then this peptide can be predicted to be useful for tolerance induction.

It is thus possible to investigate the tolerogenic capacity of a peptide by investigating whether it can bind to an MHC class I or II molecule and be presented to a T cell without further antigen processing *in vitro.*

The peptides of the present invention are apitopes (Antigen Processing-Indepent epiTOPES) in that they are capable of binding to an MHC class II molecule and stimulating a response from factor VIII specific T cells without further antigen processing. Such apitopes can be predicted to cause tolerance to FVIII, following the rule-based method described in WO 02/16410.

A peptide of the present invention may be any length that is capable of binding to an MHC class I or II molecule without further processing. Typically, the peptide of the present invention is capable of binding MHC class II.

Peptides that bind to MHC class I molecules are typically 7 to 13, more usually 8 to 10 amino acids in length. The binding of the peptide is stabilised at its two ends by contacts between atoms in the main chain of the peptide and invariant sites in the peptide-binding groove of all MHC class I molecules. There are invariant sites at both ends of the groove which bind the amino and carboxy termini of the peptide. Variations is peptide length are accomodated by a kinking in the peptide backbone, often at proline or glycine residues that allow the required flexibility.

Peptides which bind to MHC class II molecules are typically between 8 and 20 amino acids in length, more usually between 10 and 17 amino acids in length, and can be longer (for example up to 40 amino acids). These peptides lie in an extended conformation along the MHC II peptide-binding groove which (unlike the MHC class I peptide-binding groove) is open at both ends. The peptide is held in place mainly by main-chain atom contacts with conserved residues that line the peptide-binding groove.

### PEPTIDE SEQUENCES

The first aspect of the invention relates to a peptide consisting of one of the following sequences:

Various antigen processing independent presentation systems (APIPS) are known, including:
a) fixed APC (with or without antibodies to CD28);
b) Lipid membranes containing Class I or II MHC molecules (with or without antibodies to CD28); and
c) purified natural or recombinant MHC in plate-bound form (with or without antibodies to CD28).

All of these systems are capable of presenting antigen in conjunction with an MHC molecule, but are incapable of processing antigen. In all these systems the processing function is either absent or disabled. This makes it possible to investigate whether a peptide can bind to an MHC class I or II molecule and be presented to a T cell without further antigen processing.

The use of fixed APC to investigate T cell responses is well known in the art, for example in studies to investigate the minimal epitope within a polypeptide, by measuring the response to truncated peptides (Fairchild et al (1996) Int. Immunol. 8:1035-1043). APC may be fixed using, for example formaldehyde (usually paraformaldehyde) or glutaraldehyde.

Lipid membranes (which may be planar membranes or liposomes) may be prepared using artificial lipids or may be plasma membrane/microsomal fractions from APC.

In use, the APIPS may be applied to the wells of a tissue culture plate. Peptide antigens are then added and binding of the peptide to the MHC portion of the APIPS is detected by addition of selected T cell lines or clones. Activation of the T cell line or clone may be measured by any of the methods known in the art, for example via ³H-thymidine incorporation or cytokine secretion.

### FACTOR VIII

The peptide of the invention may be derivable from factor VIII.

Factor VIII participates in the intrinsic pathway of blood coagulation; factor VIII is a cofactor for factor IXa which, in the presence of Ca+2 and phospholipids, converts factor X to the activated form Xa.

The factor VIII gene produces two alternatively spliced transcripts. Transcript variant 1 encodes a large glycoprotein, isoform a, which circulates in plasma and associates with von Willebrand factor in a noncovalent complex. This protein undergoes multiple cleavage events. Transcript variant 2 encodes a putative small protein, isoform b, which consists primarily of the phospholipid binding domain offactor VIIIc. This binding domain is essential for coagulant activity.

The complete 186,000 base-pair sequence of the human factor VIII gene was elucidated in the mid 1980s (Gitschier et al (1984) Nature 312 326-330). At the same time, DNA clones encoding the complete 2351 amino acid sequence were used to produce biologically active factor VIII in cultured mammalian cells (Wood et al (1984) Nature 312:330-337). The complete 2,351 amino acid sequence for human factor VIII is given in SEQ ID No. 1.

The peptide of the present invention may be derivable from factor VIII. The peptide may, for example, consist of a contiguous sequence of amino acids from the factor VIII sequence. The peptide may be obtainable or obtained from cleavage of the factor VIII sequence.

A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

### TOLERANCE

T cell epitopes play a central role in the adaptive immune response to any antigen, whether self or foreign. The central role played by T cell epitopes in hypersensitivity diseases (which include allergy, autoimmune diseases and transplant rejection) has been demonstrated through the use of experimental models. It is possible to induce inflammatory or allergic diseases by injection of synthetic peptides (based on the structure of T cell epitopes) in combination with adjuvant.

By contrast, it has been shown to be possible to induce immunological tolerance towards particular antigens by administration of peptide epitopes in soluble form. Administration of soluble peptide antigens has been demonstrated as an effective means of inhibiting disease in experimental autoimmune encephalomyelitis (EAE - a model for multiple sclerosis (MS)) (Metzler and Wraith (1993) Int. Immunol. 5:1159-1165; Liu and Wraith (1995) Int. Immunol. 7:1255-1263; Anderton and Wraith (1998) Eur. J. Immunol. 28:1251-1261); and experimental models of arthritis, diabetes, and uveoretinitis (reviewed in Anderton and Wraith (1998) as above). This has also been demonstrated as a means of treating an ongoing disease in EAE (Anderton and Wraith (1998) as above).

Tolerance is the failure to respond to an antigen. Tolerance to self antigens is an essential feature of the immune system, when this is lost, autoimmune disease can result. The adaptive immune system must maintain the capacity to respond to an enormous variety of infectious agents while avoiding autoimmune attack of the self antigens contained within its own tissues. This is controlled to a large extent by the sensitivity of immature T lymphocytes to apoptotic cell death in the thymus (central tolerance). However, not all self antigens are detected in the thymus, so death of self-reactive thymocytes remains incomplete. There are thus also mechanisms by which tolerance may be acquired by mature self-reactive T lymphocytes in the peripheral tissues (peripheral tolerance). A review of the mechanisms of central and peripheral tolerance is given in Anderton et al (1999) (Immunological Reviews 169:123-137).

In haemophilia A; patients have a defect in the factor VIII gene. This means that factor VIII is not recognised as a "self" antigen by the immune system. When factor VIII is administered during coagulation factor replacement therapy, therefore, an alloimmune response is generated to the foreign protein, leading to the production of FVIII inhibitor antibodies.

The peptides of the present invention are capable of inducing tolerance to factor VIII such that when FVIII is administered therapeutically, it does not induce an immune response and FVIII inhibitors do not develop.

Acquired haemophilia is an autoimmune disease in which tolerance to factor VIII breaks down. In this case, peptides of the present invention may be administered to reinstate tolerance to this self protein and curtail the pathogenic immune response.

Tolerance may result from or be characterised by the induction of anergy in at least a portion of CD4+ T cells. In order to activate a T cell, a peptide must associate with a "professional" APC capable of delivering two signals to T cells. The first signal (signal 1) is delivered by the MHC-peptide complex on the cell surface of the APC and is received by the T cell via the TCR. The second signal (signal 2) is delivered by costimulatory molecules on the surface of the APC, such as CD80 and CD86, and received by CD28 on the surface of the T cell. It is thought that when a T cell receives signal 1 in the absence of signal 2, it is not activated and, in fact, becomes anergic. Anergic T cells are refractory to subsequent antigenic challenge, and may be capable of suppressing other immune responses. Anergic T cells are thought to be involved in mediating T cell tolerance.

Without wishing to be bound by theory, the present inventors predict that peptides which require processing before they can be presented in conjunction with MHC molecules do not induce tolerance because they have to be handled by mature antigen presenting cells. Mature antigen presenting cells (such as macrophages, B cells and dendritic cells) are capable of antigen processing, but also of delivering both signals 1 and 2 to a T cell, leading to T cell activation. Apitopes, on the other hand, will be able to bind class II MHC on immature APC. Thus they will be presented to T cells without costimulation, leading to T cell anergy and tolerance.

Of course, apitopes are also capable of binding to MHC molecules at the cell surface of mature APC. However, the immune system contains a greater abundance of immature than mature APC (it has been suggested that less than 10% of dendritic cells are activated, Summers et al. (2001) Am. J. Pathol. 159: 285-295). The default position to an apitope will therefore be anergy/tolerance, rather than activation.

The induction of tolerance to FVIII can be monitored in *vivo* by looking for a reduction in the level of:
(i) FVIII inhibitory antibodies:
(ii) CD4+ T cells specific for FVIII
(iii) B cells capable of secreting FVIII inhibitory antibodies
by techniques known in the art.

It has been shown that, when tolerance is induced by peptide administration, the capacity of antigen-specific CD4+ T cells to proliferate is reduced. Also, the production of IL-2, IFN-γ and IL-4 production by these cells is downregulated, but production of IL-10 is increased. Neutralisation of IL-10 in mice in a state of peptide-induced tolerance has been shown to restore completely susceptibility to disease. It has been proposed that a population of regulatory cells persist in the tolerant state which produce IL-10 and mediate immune regulation (Burkhart et al (1999) Int. Immunol. 11:1625-1634).

The induction of tolerance can therefore also be monitored by various techniques including:
(a) the induction of anergy in CD4+ T cells (which can be detected by subsequent challenge with FVIII *in vitro)*;
(b) changes in the CD4+ T cell population, including
   (i) reduction in proliferation;
   (ii) down-regulation in the production of IL-2, IFN-γ and IL-4; and
   (iii) increase in the production of IL-10.

As used herein, the term "tolerogenic" means capable of inducing tolerance.

### COMPOSITION

The present invention also relates to a composition, such as a pharmaceutical composition comprising a peptide according to the invention.

The peptide may comprise a plurality of peptides, for example, two, three, four, five or six peptides of the invention.

The peptides of the composition may each comprise a different epitope.

The composition may comprise the peptides PRCLTRYYSSFVNME and DNIMVTFRNQASRPY.

The composition of the present invention may be for prophylactic or therapeutic use.

When administered for prophylactic use, the composition may reduce or prevent the generation of an immune response to FVIII. The level of immune response is less than would have been obtained in the patient had not been treated with the composition. The term "reduce" indicates that a partial reduction in immune response is observed, such as a 50%, 70%, 80% or 90% reduction in the response that would have been observed in the patient had not been treated with the composition (or in the response observed in an untreated patient over the same time-frame). The term "prevent" indicates that no appreciable immune response to FVIII is observed.

When administered for therapeutic use, the composition may suppress an already ongoing immune response to FVIII. The term "suppress" indicates a reduction in the level of an on-going immune response, compared to the level before peptide treatment, or the level which would have been observed at the same time point had the treatment not been given.

Treatment with the composition of the present invention may cause a reduction in levels of any or all of the following:
(i) FVIII inhibitory antibodies:
(ii) CD4+ T cells specific for FVIII
(iii) B cells secreting FVIII inhibitory antibodies.

Detection of all these factors can be carried out by techniques known in the art, such as ELISA, FACS etc.

Treatment with the composition of the present invention may also or alternatively cause anergy in CD4+ T cells specific for FVIII. Anergy can be detected by for example subsequent challenge with FVIII *in vitro.*

It is important to bear in mind that not all immune responses to FVIII are pathogenic. Non-inhibitory anti-FVIII antibodies may be found in haemophilia patients without inhibitors (Moreau et al (2000) Blood 95:3435-41) and approximately 15% of healthy blood donors (Algiman *et al* (1992) 89:3795-9).

FVIII inhibitors may be detected by the Nijmegen modification of the clotting Bethesda assay, in which the ability of the patient's plasma to inactivate FVIII in normal plasma is tested. A Bethesda unit is defined as the amount of antibody that neutralizes 50% of plasma FVIII activity, and titres of 0.6BU or greater suggest the presence of antibody.

Inhibitors are generally classified as low titre if the level is <5 BU and high titre if ≥ 5 BU.

The level of circulating FVIII inhibitory antibodies may be reduced to 90%, 75%, 50%, 20%, 10% 5% of the level of antibodies which would have been observed had the patient not received treatment.

The level of circulating FVIII inhibitory antibodies may be reduced to 5, 4, 3, 2, 1 or 0.5 BU.

The peptides and composition of the invention may increase the amount or proportion of therapeutically administered FVIII which is available to aid clotting in a patient. This is due to the reduction in FVIII inhibitors which may effectively remove a proportion of FVIII from exerting its therapeutic function. The peptide or composition of the invention may increase the amount of available FVIII by, for example, 10%, 25%, 50% 75% or 100%.

The peptides and composition of the invention may thus reduce the amount of FVIII which needs to be administered to aid clotting in a patient.

### FORMULATION

The composition may by prepared as an injectable, either as liquid solution or suspension; solid form suitable for solution in, or suspension in, liquid prior to injection may also be prepared. The preparation may also be emulsified, or the peptides encapsulated in liposomes. The active ingredients may be mixed with excipients which are pharmaceutically acceptable and compatible with the active ingredient. Suitable excipients are, for example, water, saline (for example, phosphate-buffered saline), dextrose, glycerol, ethanol, or the like and combinations thereof.

In addition, if desired, the composition may contain minor amounts of auxiliary substances such as wetting or emulsifying agents and/or pH buffering agents. Buffering salts include phosphate, citrate, acetate. Hydochloric acid and/or sodium hydryoxide may be used for pH adjustment. For stabilisation, disaccharides may be used such as sucrose or trehalose.

If the composition comprises a plurality of peptides, the relative ratio of the peptides may be approximately equal. Alternatively the relative ratios of each peptide may be altered, for example, to focus the tolerogenic response on a particular sub-set of autoreactive T-cells or if it is found that one peptide works better than the others in particular HLA types.

After formulation, the composition may be incorporated into a sterile container which is then sealed and stored at a low temperature, for example 4°C, or it may be freezedried.

Conveniently the composition is prepared as a lyophilized (freeze dried) powder. Lyophilisation permits long-term storage in a stabilised form. Lyophilisation procedures are well known in the art, see for example http://www.devicelink.com/ivdt/archive/97/01/006.html. Bulking agents are commonly used prior to freeze-drying, such as mannitol, dextran or glycine.

The composition may be administered in a convenient manner such as by the oral, intravenous (where water soluble), intramuscular, subcutaneous, sublingual, intranasal, intradermal or suppository routes or implanting (e.g. using slow release molecules).

The composition may advantageously be administered via intranasal, subcutaneous or intradermal routes.

The peptide and composition of the invention may be used to treat a human subject. The subject may have haemolphilia A, in particular severe haemophilia A. The subject may be genetically deficient in FVIII. The subject may have acquired haemophilia. The subject may have inhibitory anti-FVIII antibodies.

The subject may be undergoing or about to undergo coagulant replacement therapy with FVIII.

The subject may be undergoing or about to undergo gene therapy with the FVIII gene.

The subject may be an HLA-haplotype which is associated with a predisposition to develop inhibitory anti-FVIII alloantibodies or autoantibodies. The subject may express HLA-DR2. Methods for determining the HLA haplotype of an individual are known in the art.

Typically, a physician will determine the actual dosage which will be most suitable for an individual subject and it will vary with the age, weight and response of the particular patient.

In a preferred embodiment a "dose escalation" protocol may be followed, where a plurality of doses is given to the patient in ascending concentrations. Such an approach has been used, for example, for phospholipase A2 peptides in immunotherapeutic applications against bee venom allergy (Müller et al (1998) J. Allergy Clin Immunol. 101:747-754 and Akdis et al (1998) J. Clin. Invest. 102:98-106).

### KITS

Conveniently, if the composition comprises a plurality of peptides, they may be administered together, in the form of a mixed composition or cocktail. However, there may be circumstances in which it is preferable to provide the peptides separately in the form of a kit, for simultaneous, separate, sequential or combined administration.

The kit may also comprise mixing and/or administration means (for example a vapouriser for intranasal administration; or a syringe and needle for subcutaneous/intradermal dosing). The kit may also comprise instructions for use.

The pharmaceutical composition or kit of the invention may be used to treat and/or prevent a disease.

In particular, the composition/kit may be used to treat and/or prevent haemophilia A or acquired haemophilia.

### HAEMOPHILIA A

Hemophilia A (classic hemophilia), is caused by the deficiency of Factor VIII.

Hemophilia A has an estimated incidence of 1 in 10,000 males, while hemophilia B is estimated to occur in one in 40,000 males. Approximately 1 woman in 5,000 is a carrier for hemophilia A, and 1 in 20,000 is a carrier of hemophilia B.

Hemophilia is typically divided into three classes: severe, moderate and mild, based on the level of clotting factor in the blood. In severe hemophilia, there is less than 1 percent of normal clotting factor. The degree of severity tends to be consistent from generation to generation.

Contrary to popular belief, minor cuts and wounds do not usually present a threat to hemophiliacs. Rather, the greatest danger comes from spontaneous bleeding that may occur in joints and muscles. This is most prone to occur during years of rapid growth, typically between the ages of 5 and 15 years.

Repeated spontaneous bleeding in joints may cause arthritis, and adjacent muscles become weakened. Pressure on nerves caused by the accumulation of blood may result in pain, numbness, and temporary inability to move the affected area.

Haemophilia A is usually diagnosed with a blood test to determine the effectiveness of clotting and to investigate whether the levels of clotting factors are abnormal.

The development of purified clotting factors in the 1970s, isolated from donated blood, significantly improved the long-term outlook for hemophiliacs. Mild to moderate haemophiliacs can use treatment with FVIII on an ad hoc basis, whereas severe haemophiliacs may require regular, indefinite treatment.

Previously, patients were given factor VIII concentrates pooled from thousands of plasma donations. This lead to significant problems of contamination with viral pathogens, particularly the human immunodeficiency virus and the hepatitis viruses. Monoclonal antibody purification techniques, heat inactivation, and virucidal detergent treatments have rendered plasma-derived concentrates relatively safe.

Recombinant DNA technology has now provided a series of synthetic products,such as Recombinate™ and Kogenate™. Kogenate is made using baby hamster kidney cells expressing human factor VIII. The resulting factor is highly purified, eliminating any possibility of transmission of virus from plasma.

The peptide or composition of the present invention may be administered before and/or during factor VIII replacement therapy.

Hemophilia A is an ideal disease target for gene therapy since i) it is caused by a mutations in a single identified gene, ii) a slight increase in clotting factor levels *in vivo* can convert severe hemophilia into milder disease, and iii) current replacement therapies are considered suboptimal. Also, there is a wide range of safety if there is an "overshoot" of desired level of coagulation activity.

Unfortunately, to date the promise of gene therapy as a cure for haemophilia has not been realized, primarily because of difficulties in finding a gene delivery system which is sufficiently non-immunogenic to allow for long term expression of the clotting factor.

The peptides of the present invention would also be suitable for tolerising a subject prior to gene therapy with factor VIII and/or managing FVIII inhibitor formation in a patient following gene therapy.

### ACQUIRED HAEMOPHILIA

Acquired haemophilia is characterised by the presence of autoantibody inhibitors against FVIII in individuals with previously normal coagulation. It is a rare condition, with an estimated incidence of 1-3 per million population per year. The mortality rate associated with acquired autoantibody inhibitors approaches 25% versus the substantially lower risk of death in those with alloantibodies.

Compared to alloantibody inhibitor patients, acquired hemophilia is characterized by: (1) a more severe bleeding pattern; (2) higher incidence in older population; (3) occurrence in conjunction with identifiable underlying autoimmune diseases, lymphoproliferative or solid tumor malignancies, pregnancy, and use of certain antibiotics such as penicillin and sulfonamides in approximately 50% of cases; and (4) in vitro inhibitor activity that follow a type II pharmacokinetic pattern with incomplete neutralization of the targeted clotting factor activity by the autoantibody, typically resulting in residual factor VIII levels ranging between 2%-18% in patient plasma.

The peptide or composition of the present invention may be administered to a patient with acquired haemophilia, or to a patient believed to be at risk of developing acquired haemophilia due to, for example:
i) imminent treatment with, for example penicillin or a sulfomamide
ii) progession of a tumour or other malignancy
iii) imminent or early pregnancy.

The invention will now be further described by way of Examples, which are meant to serve to assist one of ordinary skill in the art in carrying out the invention and are not intended in any way to limit the scope of the invention.

### EXAMPLES

### Example 1: Selection of HLA-DR2 Factor VIII peptides

A series of FDVIII 15mer peptides were compared using three HLA-DR binding algorithms:
SYFPEITHI (http://www.syfpeithi.de/home.htm)
ProPred (http://www.imtech.res.in/raghava/propred/) and
and IEDB (http://www.immuneepitope.org/home.do).

Peptides were selected which were predicted to be HLA-DR2-binding by more than one of the programmes and flanking sequences were designed for the predicted core residues (table 1).

**TABLE 1**

| **Peptide No** | **FVIII First AA** | **Sequence in single amino acid code** | **Also referred to hereinas:** |
|---|---|---|---|
| 1 | 2140 | GTLMVFFGNVDSSGI | GTLMV |
| 2 | 0208 | TQTLHKFILLFAVFD | TQTLH |
| 3 | 2114 | SLYISQFIIMYSLDG | SLYIS |
| 4 | 2161 | PPIIARYIRLHPTHY | PPIIA |
| 5 | 2318 | PPLLTRYLRIHPQSW | PPLLT |
| 6 | 250 | MHTVNGYVNRSLPGL | MHTVN |
| 7 | 322 | LGQFLLFCHISSHQH | LGQFL |
| 8 | 478 | DTLLIIFKNQASRPY | DTLLI |
| 9 | 545 | PRCLTRYYSSFVNME | PRCLT |
| 10 | 607 | TENIQRFLPNPAGVQ | TENIQ |
| 11 | 1788 | DNIMVTFRNQASRPY | DNIMV |
| 12 | 2322 | RYLRIHPQSWVHQIA | RYLRI |

### Example 2: Investigating the response of HLA-DR2 restricted cells from factor VIII immunised mice to peptides

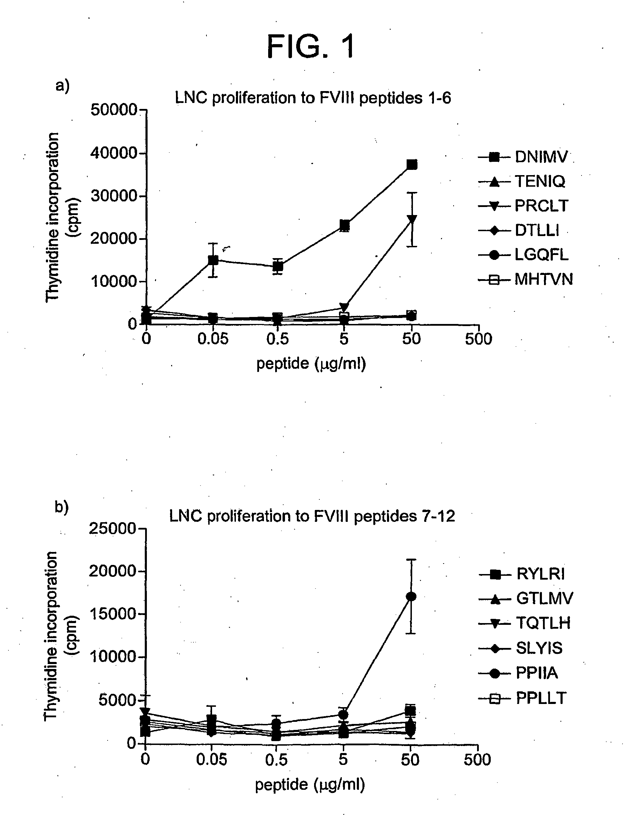

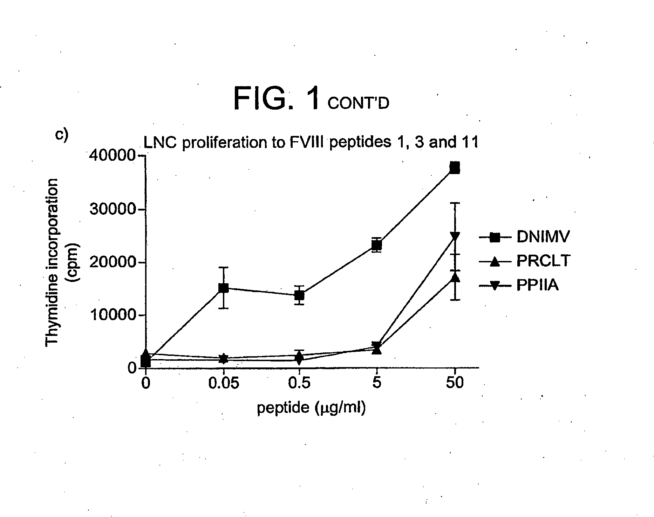

HLA-DR2 transgenic mice were immunised with human factor VIII in adjuvant. Draining lymph node cells were collected and restimulated *in vitro* with different concentrations of the 12 peptides from table 1. The results are shown in Figure 1.

HLA-DR2 restricted cells from factor VIII immunised mice clearly respond strongly to peptide DNIMV (1^{st} amino acid 1788). There are also responses to peptides PRCLT (545) and PPIIA (2161).

### Example 3: Investigating the response of T cells from HLA-DR2 mice to peptides

HLA-DR2 mice were first immunised with factor VIII in adjuvant. Spleen cells from immune mice were restimulated *in vitro* with factor VIII and the resulting lymphoblasts were fused with the BW5147 thymoma using polyethylene glycol.

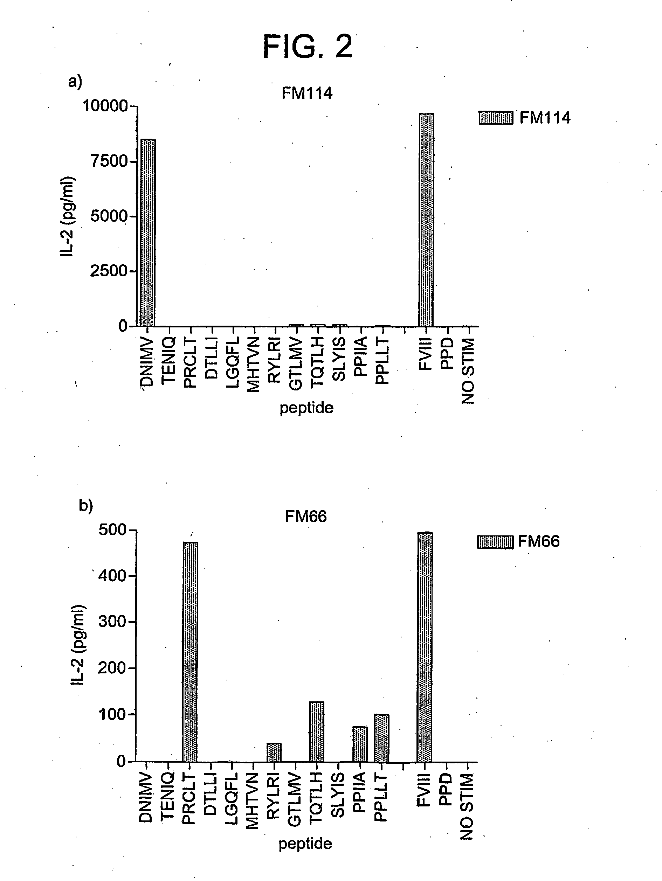

T-cell hybridomas were selected in HAT medium and the hybridomas cloned and tested for their response to factor VIII. The hybridomas were then screened for their response to the 12 predicted peptides. Of the 27 hybridomas screened, 11 responded to DNIMV, 3 to PRCLT and 3 to PPIIA, although the response to PPIIA was weaker and less specific. The response of two hybridomas specific for DNIMV and PRCLT is shown in Figure 2.

### Example 4 - Investigating the response of lymph node cells from FVIII-DR2+ mice to peptides

HLA-DR2 transgenic mice were crossed with factor VIII deficient mice to create a model of haemophilia expressing the human HLA class II MHC molecule.

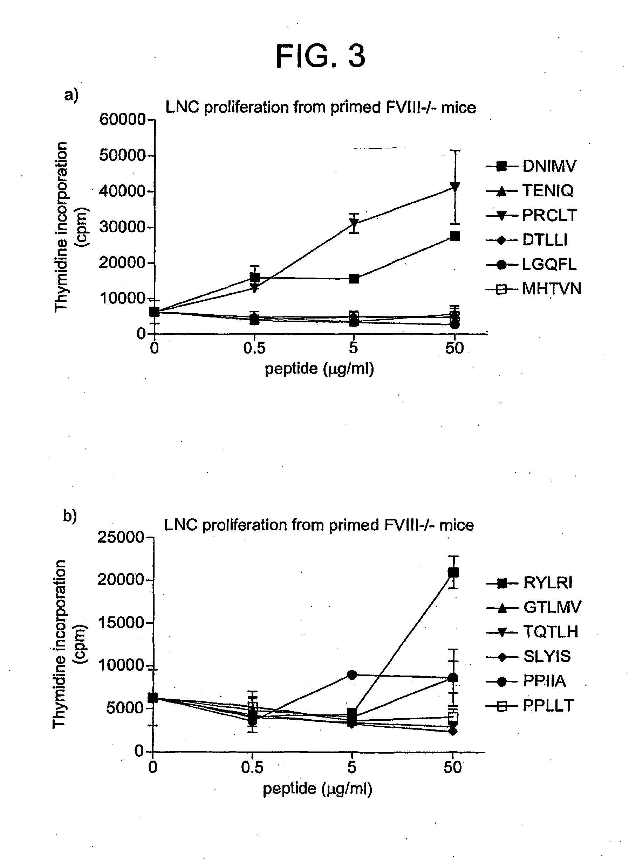

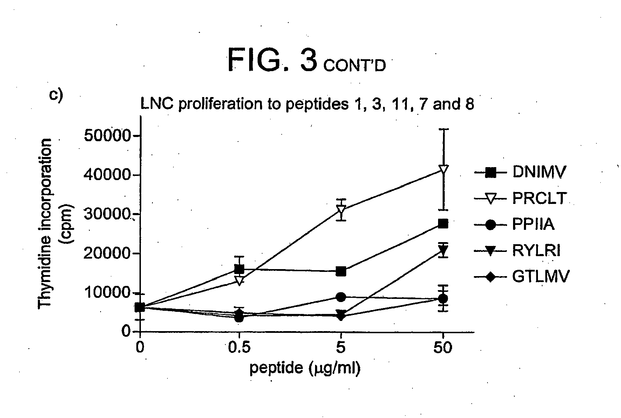

These FVIII-DR2+ animals were immunised with factor VIII in adjuvant. Draining lymph nodes were isolated and tested for their response to the peptide panel. As shown in Figure 3, these cells responded well to PRCLT and DNIMV. There was a weak response to GTLMV and significant response to RYLRI.

### Example 5 - Investigating the response of T cells from HLA-DR2 mice to peptides

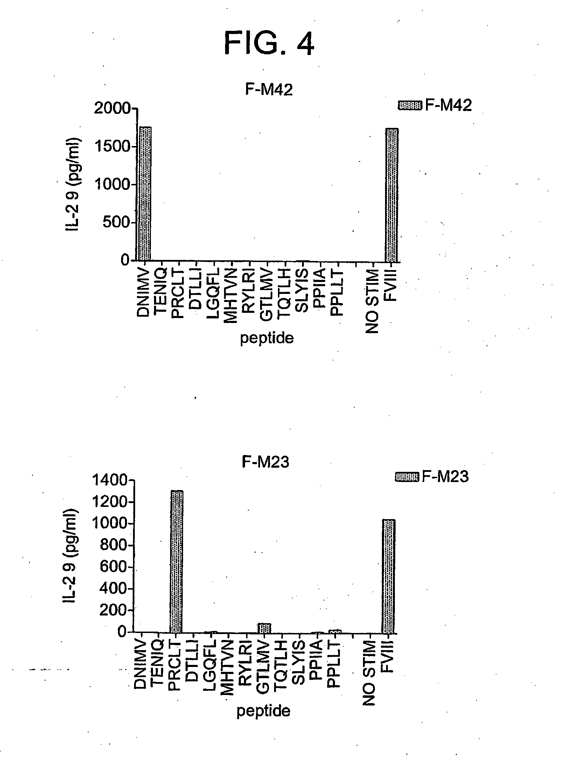

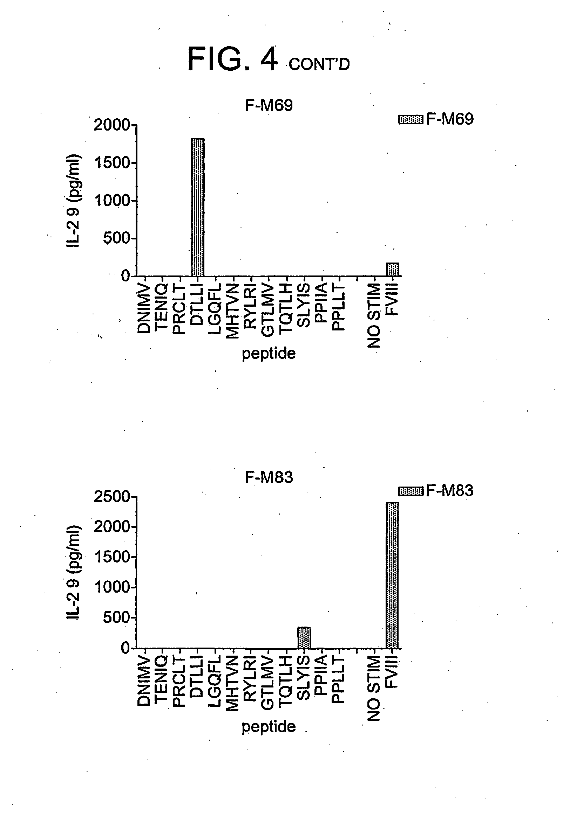

Factor VIII deficient mice expressing HLA-DR2 were immunised with factor VIII in adjuvant. Spleen cells from the immunised mice were restimulated *in vitro* with factor VIII and the resulting lymphoblasts were fused with BW5147, as described above. T-cell hybridomas were screened for their response to the 12 predicted peptides. Yet again, the majority of hybridomas responded to peptides DNIMV and PRCLT. Of 19 hybridomas specific for factor VIII, 10 responded to DNIMV, 6 to PRCLT, 1 to PPIIA, 1 to SLYIS and 1 to DTLLI. Examples of responses by these hybridomas are shown in Figure 4.

Based on these experiments it is clear that two peptides DNIMV (first amino acid number 1788) and PRCLT (first amino acid 545) constitute the immunodominant T-cell epitopes in the HLA-DR2 restricted T-cell response to human factor VIII.

### Example 6 - DNIMV and PRCLT behave as apitopes

In order to be an apitope, a peptide must be capable of binding to an MHC class I or II molecule without further antigen processing (i.e. trimming) and be presented to a T cell. In the present case, the capacity of peptides to be presented by fixed APC was investigated.

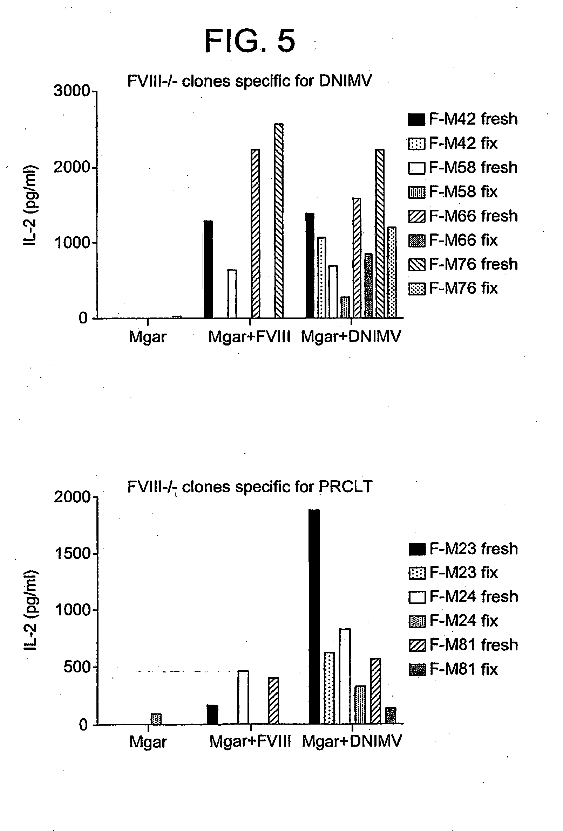

Mgar cells were either fresh or fixed with 1% paraformaldehyde. Clones were tested for antigenic specificity by culturing 100µl of hybridoma cells with 5x10⁴ Mgar cells in the presence and absence of 20µg/ml rhFVIII or peptide epitopes overnight. Supernatants were then collected and assessed for IL-2 production by ELISA. The fact that rhFVIII must be presented by live Mgar cells demonstrates that the intact protein requires antigen processing to be presented. Peptides DNIMV and PRCLT, on the other hand, are presented by both live and fixed Mgar cells indicating that these peptides function as apitopes (Figure 5).

### Example 7 - Determination of the range of peptide epitopes capable of functioning as apitopes

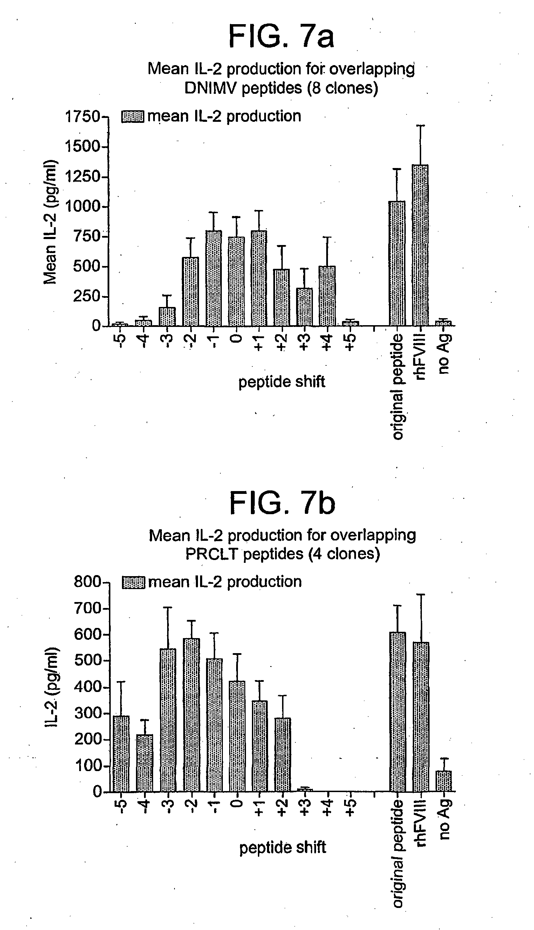

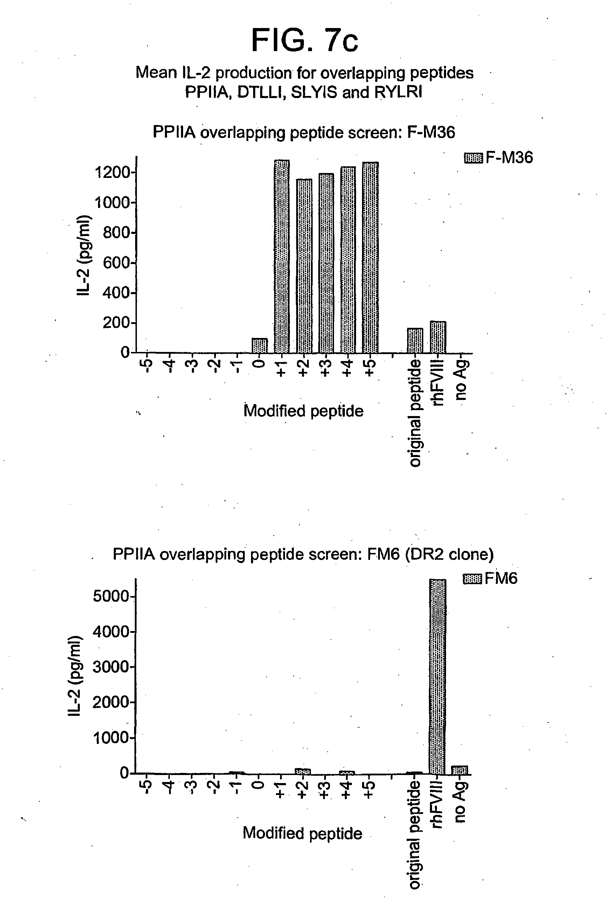

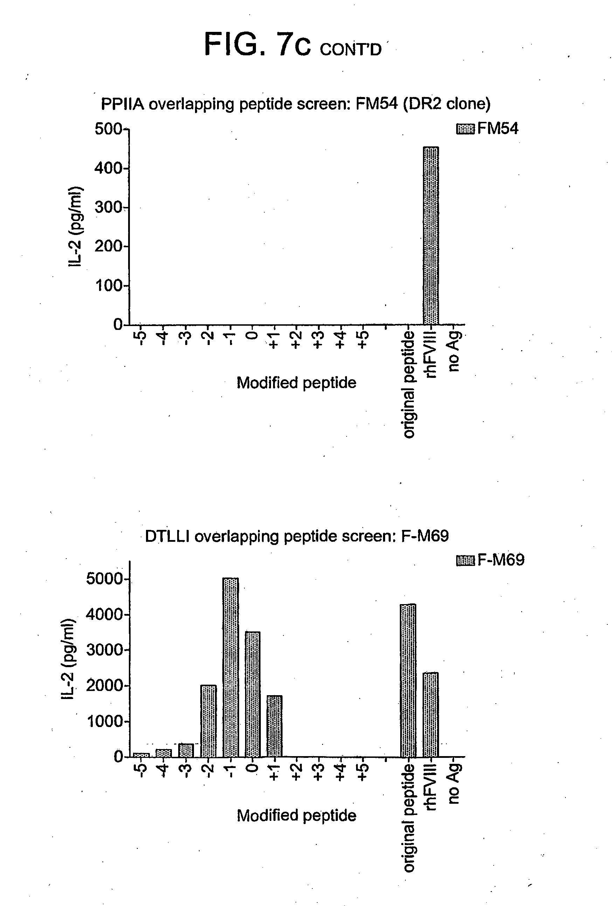

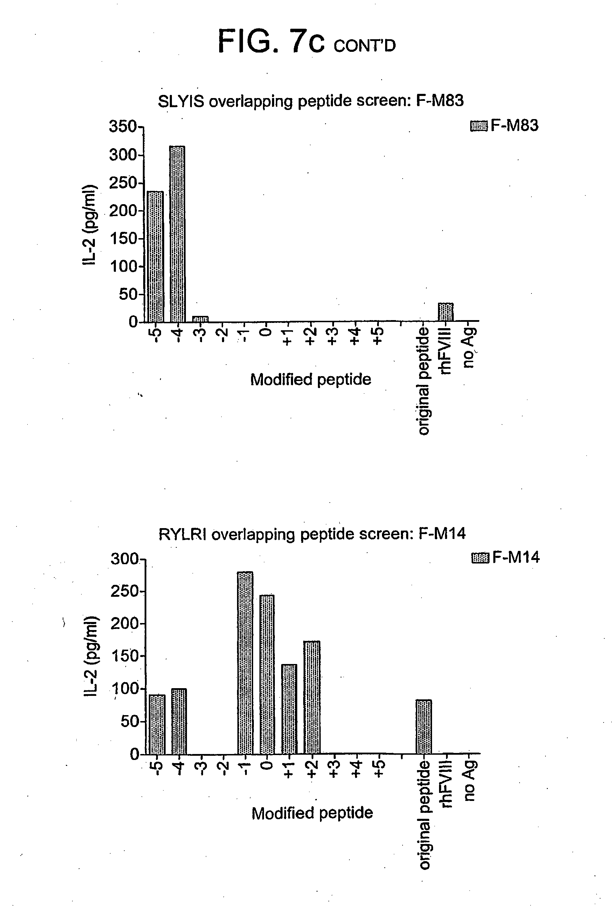

The range of peptide epitopes capable offunctioning as apitopes in the sequences surrounding DNIMV, PRCLT and the other peptides was identified by preparing panels of overlapping peptides (shown on pages 36-37) and screening these using the T-cell hybridomas using the same method as Example 5 (Figure 7).

### Example 8 - DNIMV and PRCLT induce tolerance to whole factor VIII protein

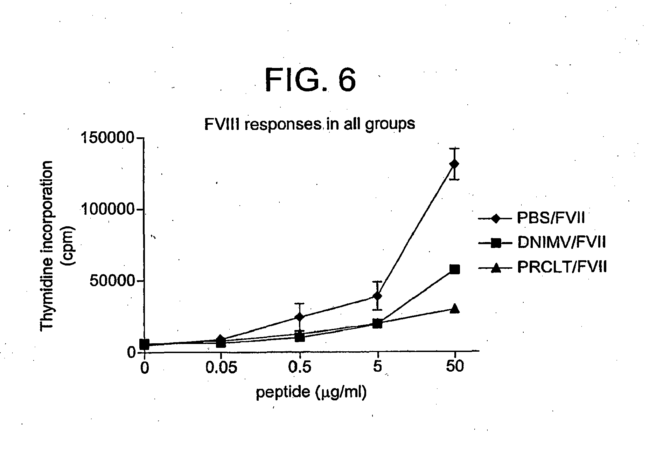

HLA-DR2 transgenic mice were treated with either of the two soluble peptides, or PBS as a control, prior to immunisation with factor VIII in adjuvant. Draining lymph nodes were isolated and the cells restimulated *in vitro* with factor VIII protein in order to assess the immune status of the mice. As shown in Figure 6, treatment of mice with either DNIMV or PRCLT led to a substantial suppression of the immune response to factor VIII.

### Example 9 - Investigation of whether DNIMV and PRCLT able to induce tolerance in the factor VIII knock out mouse

It was known from Example 8 that these two peptides are able to prevent the immune response to factor VIII in mice expressing endogenous factor VIII. The experiment was repeated with FVII I-DR2+ animals to determine whether these peptides also prevent the immune response to factor VIII in factor VIII deficient mice.

### Example 10 - Investigation of whether DNIMV and PRCLT in combination are able to induce tolerance in the factor VIII knockout mouse

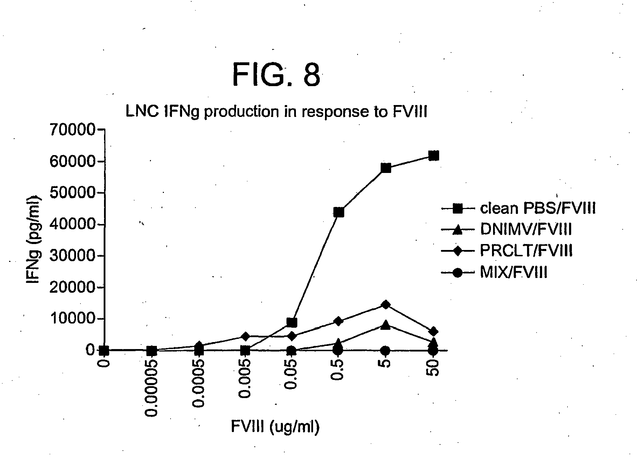

The two peptides which were shown to individually reduce the immune response to factor VIII in factor VIII deficient mice in Example 9 were combined. As shown in Figure 8, treatment of mice with both DNIMV and PRCLT led to a substantial suppression of the immune response to factor VIII, as shown by the decrease in IFN-gamma production. IFN-gamma is the major class switch lymphokine required for neutralising antibodies in the mouse. The effect demonstrated was greater than that observed using either peptide alone. '

### Methods

### (i) Recall responses for DR2+ mice primed with rhFVIII

HLA-DR2+ murine MHC class II null mice were immunised with 40µg rhFVIII emulsified in Complete Freunds Adjuvant supplemented with 400µg heat-killed *M.tuberculosis H37Ra,* subcutaneously at the base of the tail. 10 days later the mice were sacrificed and the draining lymph nodes removed. Single cell suspensions were prepared and lymphocytes incubated at 4-5x10⁵ cells per well in 96-well flat bottomed plates for 72 hours with the indicated concentrations of peptide or control antigens before pulsing with 0.5µCi/well tritiated thymidine for a further 16 hours. Plates were then frozen before cells were harvested onto glass filter mats and radioactive incorporation measured using a liquid scintillation β-counter

### (ii) FVIII peptide specificity of T cell hybridomas generated from DR2+ mice

HLA-DR2+ murine MHC class II null mice were immunised as above. On day 10 draining lymph nodes were removed and lymphocytes cultured at 2.5x10⁶ cells/ml, 1ml/well in 24 well plates in the presence of 20µg/ml rhFVIII for 3 days. Following this stimulation, lymphocytes were recovered, washed and fused with TCRα-β- BW fusion partner cells at a ratio of 4 BW cells to 1 lymphocyte, using polyethylene glycol as described by Nelson et al (1980) PNAS 77 (5):2866. Fused cells were carefully washed and then plated out in flat bottomed 96 well plates for 2 days before the addition of HAT medium to select for T cell hybridomas. Cells were monitored for growth and approximately 10 days after fusions were performed, individual clones were selected and transferred to 24 well plates in HAT medium. Clones were maintained in HAT medium for at least 2 weeks before being weaned into HT medium and then complete medium. Clones were tested for antigenic specificity by culturing 100µl of hybridoma cells with 5x10⁴ Mgar cells in the presence and absence of 20µg/ml rhFVIII overnight. Supernatants were then collected and assessed for IL-2 production by ELISA, with clones producing IL-2 in response to rhFVIII being considered positive for FVIII-specificity. To investigate the repertoire of predicted FVIII peptides FVIII-specific clones were again tested for IL-2 production, following overnight incubation with 20µg/ml of each of the 12 peptides.

### (iii) Recall responses for FVIII-/- mice primed with rhFVIII

The same method was followed as for (i), except the mice were FVIII-deficient, HLA-DR2+ and murine MHC class II null.

### (iv) FVIII peptide specificity of T cell hybridomas generated from FVIII-/- mice

The same method was followed as for (ii), except the mice were FVIII-deficient and HLA-DR2+.

### (v) Tolerisation of FVIII-specific responses in DR2+ mice by pre-treatment with Immunodominant FVIII peptides

HLA-DR2+ murine MHC class II null mice were treated 3 times with 100µg of DNIMV, PRCLT or PPIIA dissolved in PBS, or the equivalent volume of PBS alone. Peptides were administered intraperitoneally, with 3-4 days between each dose. Following the final administration, mice were primed with rhFVIII emulsified in complete Freunds adjuvant as for (i).10 days later, draining lymph nodes were recovered and lymphocytes subsequently cultured in vitro with rhFVIII, or each of the tolerising peptides as well as control antigens, for 72 hours before the addition of tritiated thymidine as for (i).

### (vi) Tolerisation of FVIII-specific responses in DR2+ mice by pre-treatment with a combination immunodominant FVIII peptides

HLA-DR2+ murine MHC class II null mice were treated 3 times with DNIMV, PRCLT or a combination of both DNIMV and PRCLT dissolved in PBS, or the equivalent volume of PBS alone. Peptides were administered intraperitoneally, over 8 days. Following the final administration, mice were primed with rhFVIII emulsified in complete Freunds adjuvant as for (i). 10 days later, draining lymph nodes were recovered and lymphocytes subsequently re-stimulated *in vitro* with rhFVIII. The supernatants were then collected and IFN-gamma was measured.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in cellular studies using flow cytometry or related fields are intended to be within the scope of the following claims.

### Overlapping peptide panels prepared in Example 7

### Overlapping set for DTLLIIFKNQASRPY

| | | |
|---|---|---|
| 1. | 473-488 | YGEVGDTLLUFKNQ |
| 2. | 474-489 | GEVGDTLLDFKNQA |
| 3. | 475-490 | EVGDTLLIIFKNQAS |
| 4. | 476-491 | VGDTLLIIFKNQASR |
| 5. | 477-492 | GDTLLIIFKNQASRP |
| 6. | 478-493 | DTLLIIFKNQASRPY |
| 7. | 479-494 | TLLIIFKNQASRPYN |
| 8. | 480-495 | LLIIFKNQASRPYNI |
| 9. | 481-496 | LIIFKNQASRPYNIY |
| 10. | 482-497 | IIFKNQASRPYNIYP |
| 11. | 483-498 | IFKNQASRPYNIYPH |

### Overlapping set for PRCLTRYYSSFVNME

| | | |
|---|---|---|
| 1. | 540-554 | PTKSDPRCLTRYYSS |
| 2. | 541-555 | TKSDPRCLTRYYSSF |
| 3. | 542-556 | KSDPRCLTRYYSSFV |
| 4. | 543-557 | SDPRCLTRYYSSFVN |
| 5. | 544-558 | DPRCLTRYYSSFVNM |
| 6. | 545-559 | PRCLTRYYSSFVNME |
| 7. | 546-560 | RCLTRYYSSFVNMER |
| 8. | 547-561 | CLTRYYSSFVNMERD |
| 9. | 548-562 | LTRYYSSFVNMERDL |
| 10. | 549-563 | TRYYSSFVNMERDLA |
| 11. | 550-564 | RYYSSFVNMERDLAS |

### Overlapping set for DNIMVTFRNQASRPY

| | | |
|---|---|---|
| 1. | 1783-1797 | RAEVEDNIMVTFRNQ |
| 2. | 1784-1798 | AEVEDNIMVTFRNQA |
| 3. | 1785-1799 | EVEDNIMVTFRNQAS |
| 4. | 1786-1800 | VEDNIMVTFRNQASR |
| 5. | 1787-1801 | EDNIMVTFRNQASRP |
| 6. | 1788-1802 | DNIMVTFRNQASRPY |
| 7. | 1789-1803 | NIMVTFRNQASRPYS |
| 8. | 1790-1804 | IMVTFRNQASRPYSF |
| 9. | 1791-1805 | MVTFRNQASRPYSFY |
| 10. | 1792-1806 | VTFRNQASRPYSFYS |
| 11. | 1793-1807 | TFRNQASRPYSFYSS |

### Overlapping set for SLYISQFIIMYSLDG

| | | |
|---|---|---|
| 1. | 2109-2123 | RQKFSSLYISQFIIM |
| 2. | 2110-2124 | QKFSSLYISQFIIMY |
| 3. | 2111-2125 | KFSSLYISQFIIMYS |
| 4. | 2112-2126 | FSSLYISQFIIMYSL |
| 5. | 2113-2127 | SSLYISQFIIMYSLD |
| 6. | 2114-2128 | SLYISQFIIMYSLDG |
| 7. | 2115-2129 | LYISQFIIMYSLDGK |
| 8. | 2116-2130 | YISQFIIMYSLDGKK |
| 9. | 2117-2131 | ISQFIIMYSLDGKKW |
| 10. | 2118-2132 | SQFIIMYSLDGKKWQ |
| 11. | 2119-2133 | QFIIMYSLDGKKWQT |

### Overlapping set for PPIIARYIRLHPTHY

| | | |
|---|---|---|
| 1. | 2156-2170 | HNIFNPPIIARYIRL |
| 2. | 2157-2171 | NIFNPPIIARYIRLH |
| 3. | 2158-2172 | IFNPPIIARYIRLHP |
| 4. | 2159-2173 | FNPPIIARYIRLHPT |
| 5. | 2160-2174 | NPPIIARYIRLHPTH |
| 6. | 2161-2175 | PPIIARYIRLHPTHY |
| 7. | 2162-2176 | PIIARYIRLHPTHYS |
| 8. | 2163-2177 | IIARYIRLHPTHYSI |
| 9. | 2164-2178 | IARYIRLHPTHYSIR |
| 10. | 2165-2179 | ARYIRLHPTHYSIRS |
| 11. | 2166-2180 | RYIRLHPTHYSIRST |

### Overlapping set for RYLRIHPQSWVHQIA

1.2317-2331 PPLLTRYLRIHPQSW 2.2318-2332 PLLTRYLRIHPQSWV 3. 2319-2333 LLTRYLRIHPQSWVH. 4. 2320-2334 LTRYLRIHPQSWVHQ 5. 2321-2335 TRYLRIHPQSWVHQI 6. 2322-2336 RYLRIHPQSWVHQIA 7. 2323-2337 YLRIHPQSWVHQIAL 8. 2324-2338 LRIHPQSWVHQIALR 9. 2325-2339 RIHPQSWVHQIALRM 10. 2326-2340 IHPQSWVHQIALRME 11. 2327-2341 HPQSWVHQIALRMEV

## Claims

1. A peptide which consists of one of the following sequences:

2. A composition comprising a plurality of peptides according to claim 1.

3. A peptide according to claim 1, or a composition according to claim 2, for use in suppressing or preventing the production of factor VIII inhibitor antibodies *in vivo*.

4. A peptide according to claim 1, or a composition according to claim 2, for use in a method for treating haemophilia in a subject which comprises the step of administration of a peptide according to claim 1, or a composition according to claim 2, to the subject.

5. A peptide or composition according to claim 4, wherein the subject has haemophilia A, and is undergoing, or is about to undergo, factor VIII replacement therapy.

6. A peptide or composition according to claim 4, wherein the subject has, or is at risk from contracting, acquired haemophilia.

7. A peptide or composition according to claim 4, wherein the subject is HLA-DR2.

## Patentansprüche

1. Peptid, welches aus den folgenden Sequenzen besteht:

2. Zusammensetzung, enthaltend eine Mehrzahl von Peptiden nach Anspruch 1.

3. Peptid nach Anspruch 1 oder Zusammensetzung nach Anspruch 2 zur Verwendung beim Unterdrücken oder Verhindern der Produktion von Faktor-VIII-Inhibitor-Antikörpern in vivo.

4. Peptid nach Anspruch 1 oder Zusammensetzung nach Anspruch 2 zur Verwendung bei einem Verfahren zur Behandlung von Hämophilie in einem Patienten, welches den Schritt der Verabreichung eines Peptids nach Anspruch 1 oder einer Zusammensetzung nach Anspruch 2 an den Patienten umfasst.

5. Peptid oder Zusammensetzung nach Anspruch 4, wobei der Patient an Hämophilie A leidet und sich einer Faktor-VIII-Ersatztherapie unterzieht oder im Begriff ist, sich einer solchen Therapie zu unterziehen.

6. Peptid oder Zusammensetzung nach Anspruch 4, wobei der Patient an erworbener Hämophilie leidet oder bei ihm das Risiko einer Erkrankung an erworbener Hämophilie besteht.

7. Peptid oder Zusammensetzung nach Anspruch 4, wobei der Patient HLA-DR2 ist.

## Revendications

1. Peptide qui est constitué par l'une des séquences suivantes :

2. Composition comprenant une pluralité de peptides selon la revendication 1.

3. Peptide, selon la revendication 1, ou composition, selon la revendication 2, destiné(e) à être utilisé(e) dans la suppression ou la prévention de la production d'anticorps inhibiteurs du facteur VIII *in vivo.*

4. Peptide, selon la revendication 1, ou composition, selon la revendication 2, destiné(e) à être utilisé(e) dans un procédé de traitement de l'hémophilie chez un sujet, qui comprend l'étape d'administration d'un peptide, selon la revendication 1, ou d'une composition, selon la revendication 2, au sujet.

5. Peptide ou composition selon la revendication 4, le sujet étant atteint d'une hémophilie A et étant soumis, ou étant sur le point d'être soumis, à une thérapie de remplacement du facteur VIII.

6. Peptide ou composition selon la revendication 4, le sujet étant atteint d'une hémophilie acquise ou étant à risque de la contracter.

7. Peptide ou composition selon la revendication 4, le sujet étant de type HLA-DR2.
